# EUROPEAN PATENT SPECIFICATION

(11) **EP 2 034 977 B1**
(45) Date of publication and mention of the grant of the patent: **13.07.2011**
(21) Application number: 07724368.1
(22) Date of filing: 19.04.2007
(51) Int. Cl.: A61K 31/00, A61K 31/485, A61Q 5/00, A61Q 5/10

(54) **USE OF OPIOID RECEPTOR ANTAGONISTS**
VERWENDUNG VON OPIOID-REZEPTOR-ANTAGONISTEN
UTILISATION D'ANTAGONISTES DU RÉCEPTEUR OPIOÏDE

(30) Priority: 21.04.2006 EP 06008276
(43) Date of publication of application: 18.03.2009
(73) Proprietor: DSM IP Assets B.V., 6411 TE Heerlen (NL)
(72) Inventor: BEUMER, Raphael, 79541 Lörrach (DE); KLOCK, Jochen, 79104 Freiburg (DE); MASSA, Daniela, 79111 Freiburg (DE); MENDROK-EDINGER, Christine, 79618 Rheinfelden-Minseln (DE); TREMBLEY, Marcella, CH-4055 Basel (CH)
(74) Representative: Schwander, Kuno
(86) International application number: PCT/EP2007/003431
(87) International publication number: WO 2007/121916

(56) References cited:
- WO-A-2007/039058
- US-A- 4 416 886
- TOBIN ET AL: "Hair melanocytes as neuro-endocrine sensors-Pigments for our imagination", MOLECULAR AND CELLULAR ENDOCRINOLOGY, ELSEVIER IRELAND LTD, IE LNKD- DOI:10.1016/J.MCE.2005.09.001, vol. 243, no. 1-2, 24 November 2005 (2005-11-24), pages 1-11, XP005158078, ISSN: 0303-7207
- GRAY RON E ET AL: "Mu opioid receptor efficacy and potency of morphine-6-glucuronide in neonatal guinea pig brainstem membranes: Comparison with transfected CHO cells", BRAIN RESEARCH BULLETIN, vol. 54, no. 5, 15 March 2001 (2001-03-15) , pages 499-505, ISSN: 0361-9230
- TOBIN ET AL: "Hair melanocytes as neuro-endocrine sensors-Pigments for our imagination", MOLECULAR AND CELLULAR ENDOCRINOLOGY, ELSEVIER IRELAND LTD, IE LNKD- DOI:10.1016/J.MCE.2005.09.001, vol. 243, no. 1-2, 24 November 2005 (2005-11-24), pages 1-11, XP005158078, ISSN: 0303-7207
- GRAY RON E ET AL: "Mu opioid receptor efficacy and potency of morphine-6-glucuronide in neonatal guinea pig brainstem membranes: Comparison with transfected CHO cells", BRAIN RESEARCH BULLETIN, vol. 54, no. 5, 15 March 2001 (2001-03-15) , pages 499-505, ISSN: 0361-9230

## Description

The present invention relates to a new use of µ-opioid receptor antagonists in hair care, particularly for the prevention of the graying of hair and/ or for restoration and/ or maintenance of the natural hair color. Furthermore, the invention relates to a method of preventing the graying of hair and/ or restoring and/ or maintaining the natural hair color which comprises administering to the scalp or hair of a person in need of such treatment a composition comprising an effective amount of a µ-opioid receptor antagonist.

US 4416886 discloses a topical treatment for relieving pruritis wherein naloxone, a pharmaceutically acceptable salt or a pharmaceutically acceptable chemical derivative is topically applied in a lotion, solution, cream or ointment.

Tobin and Kauser, (Mol. Cell. Endocrinol. (2005); 243:1-11) disclose that beta-endorphine stimulates melanogenesis in hair follicle melanocytes via µ-opioid receptor activation.

Gray et al. (Brain Research Bulletin (2001); 54:499-505) disclose the binding assays to determine inhibitory activity of naloxone on µ-opioid receptors.

Surprisingly, it has been found that antagonists of µ-opioid receptors, in contrast to their effect on epidermal melanocytes (EM) (see e.g. WO07039058), increase the intracellular melanin production in hair follicle melanocytes (HFM) and, therefore, can be used against unwanted graying of hair and loss of native hair color, respectively.

The term "antagonists of opioid receptors" as used herein denotes any compound which inhibits the opioid receptor signalling or down-regulates the expression of the opioid receptors in human skin cells in particular melanocytes but not limited to them. The opioid receptor antagonists may be opioid analogues, e.g., (CAS number given in parenthesis where appropriate) Naloxone (465-65-6); Naloxonazine (82824-01-9); Cyprodime (118111-54-9); β-Funaltrexamine (72782-05-9); Nalbuphine (20594-83-6); RX 8008M (40994-80-7); SDZ 210-096 (109026-86-0); Clocinnamox (117332-69-1); NIH 10236 (88167-37-7); BU 165 (173321-27-2); BU 164 (173429-52-2); BU 158 (173429-53-3); BU 160 (173429-56-6); BU 161 (173429-57-7); BU 162 (173429-58-8); Buprenorphine (52485-79-7); IOXY (141392-28-1); NPC 168 (115160-07-1); Naloxazone (73674-85-8); N-Methylnaloxonium Iodide (93302-47-7); 3-Methoxynaltrexone Hydrochloride; 7-Benzylidenenaltrexone 129468-28-6); Naltrindole Isothiocyanate (126876-64-0); BNTX (153611-34-8); Naltriben (111555-58-9); Naltrexone (16590-41-3); Nalmefene (55096-26-9); β-Chlornaltrexamine (67025-94-9); Diprenorphine (14357-78-9); nor-Binaltorphimine (105618-27-7); Naltrindole (111555-53-4); or (poly)peptides, e.g., CTAP (103429-32-9); TCTOP (115981-70-9); TCTAP (115981-71-0); CTOP (103429-31-8); Tyr MIF-1 (77133-61-0); CCK-8 (25126-32-3); CG 3703 (90243-66-6); compounds disclosed in Peptide Research 1995, 8(3), 124-37, Proceedings of the National Academy of Sciences of the United States of America (1993), 90(22), 10811-15, Regulatory Peptides (1994), (Suppl. 1), S53-S54; SMS 201-995 (83150-76-9); e-PMTC as disclosed in Medicinal Chemistry Research (1994), 4(4), 245-53; CTP (103335-28-0); TIPP (146369-65-5); ICI 154129 (83420-94-4); ICI 174864 (89352-67-0); or piperidine derivatives, e.g., the compounds disclosed in J. Med. Chem. 1993, 36(20); 2833-41, EP 657428 and EP 506478; or may belong to different structures, such as Quadazocine (71276-43-2); Flumazenil (78755-81-4); BIT (85951-65-1); Dezocine (53648-55-8); Ciramadol (63269-31-8). Ginseng root extract like in Journal of Ethnopharmacology (1994), 42(1), 45-51; Rimcazole (75859-04-0); MR 2266 (56649-76-4); and WIN 44441-3 (71276-44-3).

Further opioid receptor antagonists, particularly the µ-receptor, and assays for determining their efficacy in binding to the receptors have been disclosed in e.g., WO 02/098422, US 5,270,328, US 2001/0036951 A, WO 01/42207, WO 01/37785, WO 01/41705, WO 03/101963, WO 2004/005294, WO 2004/014310, WO 2004/038005, and WO 2004/051264.

Typical µ-opioid receptor antagonists for use herein include, but are not limited to, Naloxone; Naloxonazine; piperidine derivatives such as quoted above; Cyprodime; β-Funaltrexamine, Nalbuphine, CTAP, TCTOP, TCTAP, CTOP, Quadazocine, Flumazenil, RX 8008M, SDZ 210-096, Tyr MIF-1, CCK-8, CG 3703, Clocinnamox, peptides such as disclosed in Peptide Research 1995, 8(3), 124-37, Proceedings of the National Academy of Sciences of the United States of America (1993), 90(22), 10811-15, Regulatory Peptides (1994), (Suppl. 1), S53-S54; NIH 10236, BU 165, BU 164, BU 158, BU 160, BU 161, BU 162, Buprenorphine, IOXY, SMS 201-995, e-PMTC as disclosed in Medicinal Chemistry Research (1994), 4(4), 245-53; CTP,BIT, NPC 168, Naloxazone, Dezocine and Ciramadol. Other, typical κ-, δ- receptor or non-selective (still binding to µ-receptors as well) antagonists for use herein include, but are not limited to: N-Methylnaloxonium Iodide, 3-Methoxynaltrexone Hydrochloride; 7-Benzylidenenaltrexone, Ginseng root extract as disclosed in Journal of Ethnopharmacology (1994), 42(1), 45-51; Rimcazole, Naltrindole Isothiocyanate, BNTX, TIPP, Naltriben, Naltrexone, ICI 154129, MR 2266, WIN 44441-3, Nalmefene, β-Chlornaltrexamine, ICI 174864, Diprenorphine, nor-Binaltorphimine and Naltrindole.

Further µ-opioid receptor antagonists which have been detected during the screening as outlined in the examples and which can be used for the purposes of the present invention comprise Epigallocatechin 3,5-Digallate (37484-73-4), Irigenol Hexaacetate (103652-04-6), Irigenol ex Iris spp (4935-93-7), Berbamine Hydrochloride (5956-76-3), Quercetagetin (90-18-6), Acetylshikonin (24502-78-1), 2',3',4',3,4-Pentahydroxychalcone (484-76-4), beta,beta-Dimethylacryl shikonin (24502-79-2), 2,3-Dimethoxy-5-methyl-1,4-benzoquinone (605-94-7), 2,3-Dimethoxy-5-methylhydroquinone (3066-90-8), 2,3-Dimethoxy-1,4-benzoquinone (3117-02-0), 2,3-Dimethoxyhydroquinone (52643-52-4), Delphinidin chloride (528-53-0), Aureusidin (38216-54-5), Isocembrol (25269-17-4) and Robinetin (490-31-3) without being limited thereto.

Further µ-opioid receptor antagonists which can be used for the purposes of the present invention are disclosed in JP 63290897, US 4906655, WO 9302707, CA 2064373, US 5270220, US 5352680, WO 9504734, WO 9513071, EP 657428, WO 9606855, WO 9640208, US 5641861, WO 9733174, DE 19622866, US 5919897, US 5948807, WO 9945925, WO 2000008027, WO 2001037785, WO 2001041705, WO 2001042207, WO 2001046198, WO 2001068080, US 2001036951, WO 2002053533, WO 2003020277, WO 2003035622, WO 2003035645, WO 2003066050, WO 2003101963, WO 2004014310, WO 2004026305, WO 2004033458, US 2004186135, WO 2004080968, WO 2004080996, US 2004204445, WO 2004091593, WO 2004099194, US 2004254156, WO 2005003131, WO 2005030722.

The above-identified µ-opioid receptor antagonists can suitably be used in the form of physiologically acceptable salts, with inorganic acids, e.g. hydrochlorides, hydrobromides, sulfates, phosphates, or organic acids, e.g. methanesulfonates, p-toluenesulfonates, carbonates, formats, acetates, oxalates, lactates; or as hydrates as appropriate.

The above-identified µ-opioid receptor antagonists or their salts may be used as racemates or as pure enantiomers, or diastereomers or mixtures thereof. Preferably, pure enantiomers are used. If one chiral center is present, the optical purity of the mixture is preferably ≥ 80% ee, more preferably ≥ 90% ee, most preferably ≥ 95% ee. If two or more chiral centers are present the purity of the mixture is preferably ≥ 80% de, more preferably ≥ 90% de, most preferably ≥ 95% de.

Further, derivatives of these compounds as appropriate, such as esters, amides, nitriles, oximes, imines, hydrazones, ethers, acetals, semiacetals may also find use.

In all embodiments of the present invention the µ-opioid receptor antagonist is preferably selected from an opioid analogue, a peptide or polypeptide, or a piperidine, in particular an opioid analogue.

In all embodiments of the invention the µ-opioid receptor antagonist is most preferably selected from an opioid analoga such as naloxone, naloxonazine. Further preferred µ-opioid receptor antagonists are isocembrol, 2',3',4',3,4-pentahydroxychalcone, aureusidin or 2,3-dimethoxy-5-methylhydroquinone. In all embodiments of the invention particularly preferred are the a µ-opioid receptor antagonists naloxone, isocembrol, 2',3',4',3,4-pentahydroxychalcone, aureusidin and 2,3-dimethoxy-5-methylhydroquinone.

Particularly, the activity of an agent in blocking a µ-opioid receptor can be further determined by measuring the efficacy of the agent to cause a substantial increase of the total intracellular melanin production in human primary follicular melanocytes. An increase of the total intracellular melanin production of at least 20% is regarded to be indicative for usefulness of an agent for stimulating melanin formation and for increasing hair pigmentation when applied as a topical composition.

The effect of µ-opioid antagonists on melanin production in hair follicular melanocytes can be measured in a cell-based in vitro assay.

Melanocytes are isolated and cultured from hair follicles derived from a normal adult scalp biopsy. Follicular melanocytes are passaged in T25 plastic culture flasks to maximum 4^{th} to maximize in vivo-like behavior. Cells are seeded into flasks and after 2 days are incubated with test substances added to the cell growth medium. The experimental set-up includes flasks in which the cells are either not treated (control) or incubated for 4 days with the appropriate amount of µ-opioid antagonists, to assess the effect on melanin production.

After 4 days incubation, control and treated cells are collected and melanin is extracted from lysed cells. Melanin is quantified by measuring the spectrophotometric OD at 475 nm and calculating against a standardized curve. The resulting value is standardized against the number of cells counted per treatment and is given as micrograms of melanin per million cells.

In accordance with the present invention, the antagonists of µ-opioid receptors with the definitions and preferences as given above can be used in hair care compositions which contain at least one antagonist of a µ-opioid receptor, and carriers and/ or excipients or diluents conventionally used in hair care compositions.

The term "hair care composition" as used herein denotes any conventional hair care product, such as shampoos, conditioners, treatments, tonics, styling gels, mousses, hair sprays, pomades, setting lotions, coloring and permanent waving compositions. Of particular interest for the purpose of the present invention are shampoos, tonics, conditioners and treatments which may be in the form of a gel, a lotion, a tincture, a spray, a mousse, a cleansing composition or a foam and which may be applied according to individual needs, e.g., once daily as a lotion, tincture, mousse or spray; or once or twice weekly as a shampoo, conditioner or treatment.

In accordance with the invention, the µ-opioid receptor antagonists may be used in the manufacture of hair care compositions wherein they are present in an amount up to about 20 wt. %, preferably in an amount of at least about 0.0005 wt. % ; or about 0.001 wt. % or about 0.005 wt.-%. of µ-opioid receptor antagonist based on the total weight of the composition. The effective amount of a given µ-opioid receptor antagonist may be determined in test procedures such as described herein.

The hair care compositions, besides comprising a µ-opioid receptor antagonist may contain ingredients which are conventionally used in hair care compositions to prevent the graying of hair and/ or restore or maintain the natural hair color, such as 5,6-dihydroxyindoline HBr, 5,6-dihydroxyindoline HBr in combination with 2-methylresorcinol and/ or arginine.

The efficacy of the hair care compositions in restoration of the natural hair color can be shown, e.g., by procedures described below:
As a reference (control) a hair tress containing approximately 100 hairs is cut neatly above the scalp. The color of the hair within the tress is measured from the near-root part to the tip. This could be either done 1) visually (scoring) 2) high density photo documentation and analysis 3) pigment analysis and determination of the melanin content by i.e. by a) photometric means or b) by chemical reaction (i.e. formation of pyrrole-2,3,5 tricarboxy lic acid for eumelanin and aminohydroxyphenylalanine isomers for pheomelanin) and analysis by chromatographic, spectroscopic or spectrophotometric methods. For measuring the melanin content the hair has to be degraded.

A sample of the hair care composition (2-10 mL or mg/ cm², depending on the type of formulation; preferably a leave-on product such as hair tonic, lotion or cream) containing preferred amount of the µ-opioid receptor antagonist (active compound) is then applied at least once a day on the scalp, typically from 1 to 4 times daily for at least three months, especially six months (because normal hair grow rate is about 1 cm/ month) and distributed equally with a massage on the scalp. The product should not be washed out after application. When the treatment is terminated, a second hair sample is taken from the same place on the scalp and analyzed as described above.

A comparison of the melanin content, hair color or degree of graying is made intra-individually before and after application.

It is known that hair pigmentation is coupled to the hair cycle. If it is assumed that the described repigmentation process is linked or somehow correlated to hair growth cycle a phototrichogram (digital imaging) could be done in parallel with measurement of the following parameters: hair density, telogen percentage, hair diameter, growth rate to obtain additional parameters which could be indicative for repigmentation.

The hair care compositions suitably contain auxiliary agents which are conventionally used in hair care compositions such as disclosed in general terms in Ullmann's Encyclopedia of Industrial Chemistry (1989), Vol A 12, Hair Preparations, and more specifically, e.g., in International Patent Application No. WO 00/06094, WO 00/07550 and WO 01/06994.

Thus, the hair care compositions may contain further ingredients to protect the hair against detrimental environmental impact and to improve the health of the hair.

The hair care compositions may comprise additional cosmetic or dermatological adjuvants and/ or additives (cosmetic carrier) which are preferable selected from
1.) Water
2.) Water soluble organic solvents, preferably C1-C4-Alkanols
3.) Oils, fatty substances, waxes
4.) Various esters different to 3) of C6-C30 monocarboxylic acids with mono-, di-, or trivalent alcohols
5.) Saturated acyclic and cyclic hydrocarbons
6.) Fatty acids
7.) Fatty alcohols
8.) Silicone oils
9.) Surface active ingredients
and mixtures thereof.

The hair care compositions can contain further adjuvants and additives such as preservatives/ antioxidants, fatty substances/ oils, water, organic solvents, silicones, thickeners, softeners, anionic, cationic, nonionic or amphoteric emulsifiers, light screening agents, antifoaming agents, moisturizers, fragrances, surfactants, fillers, sequestering agents, anionic, cationic, nonionic or amphoteric polymers or mixtures thereof, propellants, acidifying or basifying agents, dyes, colorants, pigments or nanopigments, light stabilizers, insect repellents, antibacterial agents, preservatives or any other ingredients usually formulated into hair care compositions. The necessary amounts of the adjuvants and additives can, based on the desired product, easily be chosen by a skilled artisan in this field and will be illustrated in the examples, without being limited hereto.

### Light screening agents

Light screening agents are advantageously selected from UV-A, UV-B, UV-C and/ or broadband filters and Infrared light. Examples of UV-B or broad spectrum screening agents, i.e. substances having absorption maximums between about 290 and 340 nm may be organic or inorganic compounds. Organic UV-B or broadband screening agents are e.g. acrylates such as 2-ethylhexyl 2-cyano-3,3-diphenylacrylate (octocrylene, PARSOL^{®} 340), ethyl 2-cyano-3,3-diphenylacrylate and the like; camphor derivatives such as 4-methyl benzylidene camphor (PARSOL^{®} 5000), 3-benzylidene camphor, camphor benzalkonium methosulfate, polyacrylamidomethyl benzylidene camphor, sulfo benzylidene camphor, sulphomethyl benzylidene camphor, therephthalidene dicamphor sulfonic acid and the like; Cinnamate derivatives such as ethylhexyl methoxycinnamate (PARSOL^{®} MCX), ethoxyethyl methoxycinnamate, diethanolamine methoxycinnamate (PARSOL^{®} Hydro), isoamyl methoxycinnamate and the like as well as cinnamic acid derivatives bond to siloxanes; p-aminobenzoic acid derivatives, such as p-aminobenzoic acid, 2-ethylhexyl p-dimethylaminobenzoate, N-oxypropylenated ethyl p-aminobenzoate, glyceryl p-aminobenzoate; benzophenones such as benzophenone-3, benzophenone-4,2,2',4,4'-tetrahydroxy-benzophenone, 2,2'-dihydroxy-4,4'-dimethoxybenzophenone and the like; esters of benzalmalonic acid such as di-(2-ethylhexyl) 4-methoxybenzalmalonate; esters of 2-(4-ethoxy-anilinomethylene)propandioic acid such as 2-(4-ethoxy anilinomethylene) propandioic acid diethyl ester as described in the European Patent Publication EP 0895 776; organosiloxane compounds containing benzmalonate groups as described in the European Patent Publications EP 0358584 B1, EP 0538431 B1 and EP 0709080 A1 such as polysilicone-15 (PARSOL^{®} SLX); drometrizole trisiloxane (Mexoryl XL); imidazole derivatives such as e.g. 2-phenyl benzimidazole sulfonic acid and its salts (PARSOL^{®}HS). Salts of 2-phenyl benzimidazole sulfonic acid are e.g. alkali salts such as sodium- or potassium salts, ammonium salts, morpholine salts, salts of prim., sec. and tert. amines like monoethanol amine salts, diethanol amine salts and the like; salicylate derivatives such as isopropylbenzyl salicylate, benzyl salicylate, butyl salicylate, ethylhexyl salicylate (PARSOL^{®} EHS, NEO Heliopan OS), isooctyl salicylate or homomenthyl salicylate (homosalate, PARSOL^{®} HMS, NEO Heliopan OS) and the like; triazine derivatives such as ethylhexyl triazone (Uvinul T-150), diethylhexyl butamido triazone (Uvasorb HEB). Encapsulated UV-filters such as encapsulated ethylhexyl methoxycinnamate (Eusolex UV-pearls) or microcapsules loaded with UV-filters as e.g. disclosed in EP 1471995 and the like. Inorganic compounds are pigments such as microparticulated TiO₂, ZnO and the like. The term "microparticulated" refers to a particle size from about 5 nm to about 200 nm, particularly from about 15 nm to about 100 nm. The TiO₂ particles may also be coated by metal oxides such as e.g. aluminum or zirconium oxides or by organic coatings such as e.g. polyols, methicone, aluminum stearate, alkyl silane. Such coatings are well known in the art.

Examples of broad spectrum or UV A screening agents i.e. substances having absorption maximums between about 320 and 400 nm may be organic or inorganic compounds e.g. dibenzoylmethane derivatives such as 4-tert. butyl-4'-methoxydibenzoyl-methane (PARSOL^{®} 1789), dimethoxydibenzoylmethane, isopropyldibenzoylmethane and the like; benzotriazole derivatives such as 2,2'-methylene-bis-(6-(2H-benzotriazole-2-yl)-4-(1,1,3,3,-tetramethylbutyl)-phenol (TINOSORB M) and the like; bis-ethylhexyloxyphenol methoxyphenyl triazine (Tinosorb S) and the like; phenylene-1,4-bis-benzimidazolsulfonic acids or salts such as 2,2-(1,4-phenylene)bis-(1H-benzimidazol-4,6-disulfonic acid) (Neoheliopan AP); amino substituted hydroxybenzophenones such as 2-(4-diethylamino-2-hydroxy-benzoyl)-benzoic acid hexylester (Uvinul A plus) as described in the European Patent Publication EP 1046391; Ionic UV-A filters as described in the International Patent Publication WO2005080341 A1. Pigments such as microparticulated ZnO or TiO₂ and the like. The term "microparticulated" refers to a particle size from about 5 nm to about 200 nm, particularly from about 15 nm to about 100 nm. The particles may also be coated by other metal oxides such as e.g. aluminum or zirconium oxides or by organic coatings such as e.g. polyols, methicone, aluminum stearate, alkyl silane. Such coatings are well known in the art.

As dibenzoylmethane derivatives have limited photostability it may be desirable to photostabilize these UV-A screening agents. Thus, the term "conventional UV-A screening agent" also refers to dibenzoylmethane derivatives such as e.g. PARSOL^{®} 1789 stabilized by, e.g. 3,3-Diphenylacrylate derivatives as described in the European Patent Publications EP 0 514 491 B1 and EP 0 780 119 A1; Benzylidene camphor derivatives as described in the US Patent No. 5,605,680; Organosiloxanes containing benzmalonate groups as described in the European Patent Publications EP 0358584 B1, EP 0538431 B1 and EP 0709080 A1.

### Antioxidants

Based on the invention all known antioxidants usually formulated into hair care compositions can be used. Especially preferred are antioxidants chosen from the group consisting of amino acids (e.g. glycine, histidine, tyrosine, tryptophan) and their derivatives, imidazole (e.g. urocanic acid) and derivatives, peptides such as D,L-carnosine, D-carnosine, L-carnosine and derivatives (e.g. anserine), carotenoids, carotenes (e.g. α-carotene, β-carotene, lycopene) and derivatives, chlorogenic acid and derivatives, lipoic acid and derivatives (e.g. dihydrolipoic acid), aurothioglucose, propylthiouracil and other thiols (e.g. thioredoxine, glutathione, cysteine, cystine, cystamine and its glycosyl-, N-acetyl-, methyl-, ethyl-, propyl-, amyl-, butyl- and lauryl-, palmitoyl-; oleyl-, γ-linoleyl-, cholesteryl- and glycerylester) and the salts thereof, dilaurylthiodipropionate, distearylthiodipropionate, thiodipropionic acid and its derivatives (ester, ether, peptides, lipids, nucleotides, nucleosides and salts) as well as sulfoximine compounds (such as buthioninsulfoximine, homocysteinesulfoximine, buthioninsulfone, penta-, hexa-, heptathioninsulfoximine) in very low compatible doses (e.g. pmol to µmol/ kg), additionally (metal)-chelators (such as α-hydroxyfatty acids, palmic-, phytinic acid, lactoferrin), β-hydroxyacids (such as citric acid, lactic acid, malic acid), huminic acid, gallic acid, gallic extracts, bilirubin, biliverdin, EDTA, EGTA and its derivatives, unsaturated fatty acids and their derivatives (such as γ-linoleic acid, linolic acid, oleic acid), folic acid and its derivatives, ubiquinone and ubiquinol and their derivatives, vitamin C and derivatives (such as ascorbylpalmitate and ascorbyltetraisopalmitate, Mg-ascorbylphosphate, Na-ascorbylphosphate, ascorbyl-acetate), tocopherol and derivates (such as vitamin-E-acetate), mixtures of nat. vitamin E, vitamin A and derivatives (vitamin-A-palmitate and -acetate) as well as coniferylbenzoate, rutinic acid and derivatives, α-glycosylrutin, ferulic acid, furfurylideneglucitol, carnosine, butylhydroxytoluene, butylhydroxyanisole, trihydroxybutyrophenone, urea and its derivatives, mannose and derivatives, zinc and derivatives (e.g. ZnO, ZnSO₄), selen and derivatives (e.g. selenomethionin), stilbenes and derivatives (such as stilbenoxide, trans-stilbenoxide) and suitable derivatives (salts, esters, ethers, sugars, nucleotides, nucleosides, peptides and lipids) of the named active ingredients. One or more preservatives/ antioxidants may be present in an amount of at least 0.01 wt. % of the total weight of the composition. Preferably about 0.01 wt. % to about 10 wt. % of the total weight of the composition of the present invention is present. Most preferred, one or more preservatives/ antioxidants are present in an amount about 0.1 wt. % to about 1 wt. %.

### Surface active ingredients

Typically hair care compositions also contain surface active ingredients like emulsifiers, solubilizers and the like. An emulsifier enables two or more immiscible components to be combined homogeneously. Moreover, the emulsifier acts to stabilize the composition. Emulsifiers that may be used in the present invention in order to form O/W, W/O, O/W/O or W/O/W emulsions/ microemulsions include sorbitan oleate, sorbitan sesquioleate, sorbitan isostearate, sorbitan trioleate, polyglyceryl-3-diisostearate, polyglycerol esters of oleic/isostearic acid, polyglyceryl-6 hexaricinolate, polyglyceryl-4-oleate, polygylceryl-4 oleate/ PEG-8 propylene glycol cocoate, oleamide DEA, TEA myristate, TEA stearate, magnesium stearate, sodium stearate, potassium laurate, potassium ricinoleate, sodium cocoate, sodium tallowate, potassium castorate, sodium oleate, and mixtures thereof. Further exemplary emulsifiers are phosphate esters and the salts thereof such as cetyl phosphate (Amphisol^{®} A), diethanolamine cetyl phosphate (Amphisol^{®}), potassium cetyl phosphate (Amphisol^{®} K), sodium glyceryl oleate phosphate, hydrogenated vegetable glycerides phosphate and mixtures thereof. Furthermore, one or more synthetic polymers may be used as an emulsifier. For example, PVP eicosene copolymer, acrylates/ C₁₀₋₃₀ alkyl acrylate crosspolymer, acrylates/ steareth-20 methacrylate copolymer, PEG-22/ dodecyl glycol copolymer, PEG-45/ dodecyl glycol copolymer, and mixtures thereof. Further exemplary emulsifiers are fatty alcohols, e.g. cetearyl alcohol (Lanette O, Cognis Cooperation), cetyl alcohol (Lanette 16, Cognis Cooperation), stearyl alcohol (Lanette 18, Cognis Cooperation), Laneth-5 (Polychol 5, Croda Chemicals), furthermore sucrose and glucose derivatives, e.g. sucrose distearate (Crodesta F-10, Croda Chemicals), Methyl glucose isostearate (Isolan IS, Degussa Care Chemicals), furthermore ethoxylated carboxylic acids or polyethyleneglycol esters and polyethyleneglycol ethers, e.g. steareth-2 (Brij 72, Uniqema), steareth-21 (Brij 721, Uniqema), ceteareth-25 (Cremophor A25, BASF Cooperation), PEG-40 hydrogenated castor oil (Cremophor RH-40, BASF Cooperation), PEG-7 hydrogenated castor oil (Cremophor WO7, BASF Cooperation), PEG-30 Dipolyhydroxystearate (Arlacel P 135, Uniqema), furthermore glyceryl esters and polyglyceryl esters, e.g. polyglyceryl-3-diisostearate (Hostacerin TGI, Clariant Cooperation), polyglyceryl-2 dipolyhydroxystearate (Dehymuls PGPH, Cognis Cooperation), polyglyceryl-3 methylglucose distearate (Tego Care 450, Degussa Care Chemicals). The preferred emulsifiers are cetyl phosphate (Amphisol^{®} A), diethanolamine cetyl phosphate (Amphisol^{®}), potassium cetyl phosphate (Amphisol^{®} K), PVP Eicosene copolymer, acrylates/ C₁₀₋₃₀-alkyl acrylate crosspolymer, PEG-20 sorbitan isostearate, sorbitan isostearate, and mixtures thereof. The one or more emulsifiers are present in a total amount of at least 0.01 wt. % of the total weight of the composition. Preferably about 0.01 wt. % to about 20 wt. % of the total weight of the composition of the present invention is used. Most preferred, about 0.1 wt. % to about 10 wt. % of emulsifiers are used.

Typically hair care compositions also contain anionic, neutral, amphoteric or cationic tensides.

Exemplary anionic tensides comprise alkylsulfate, alkylethersulfate, alkylsulfonate, alkylarylsulfonate, alkylsuccinate, alkylsulfosuccinate, N-alkoylsarkosinate, acyltaurate, acylisethionate, alkylphosphate, alkyletherphosphate, alkylethercarboxylate, alpha-olefinsulfonate, especially the alkali-und earth alkali salts, e.g. sodium, potassium, magnesium, calcium, as well as ammonium- and triethanol amine-salts. The alkylethersulfate, alkyletherphosphate and alkylethercarboxylate may comprise between 1 to 10 ethylenoxide or propylenoxide units, preferably 1 to 3 ethylenoxide-units per molecule.

Suitable are e.g. sodium laurylsulfate, ammonium lauryl sulfate, sodium laurylethersulfate, ammonium laurylethersulfate, sodium lauroylsarkonisate, sodiumoleylsuccinate, ammonium laurylsulfosuccinate, sodium dodecylbenzolsulfonate, triethanolamidodecylbenzolsulfonate.

Suitable amphoteric tensides are e.g. alkylbetaine, alkylamidopropylbetaine, alkylsulfobetaine, alkylglycinate, alkylcarboxyglycinate, alkylamphoacetate or propionate, alkylamphodiacetate or dipropionate such as cocodimethylsuffopropylbetaine, laurylbetaine, cocamidopropylbetaine or sodium cocamphopropionate.

Examples of non ionic tensides are e.g. reaction products of aliphatic alcohols or alkylphenols with 6 to 20 C-Atoms of a linear or branched alkyl chain with ethyleneoxide and/ or propyleneoxide. The amount of alkyleneoxide is about 6 to 60 mole to one mol alcohol. Furthermore alkylaminoxide, mono- or dialkylalkanolamide, fatty esters of polyethylene glycols, alkylpolyglycosides or sorbitanether ester are suitable for the incorporation of hair care compositions according to the invention.

Furthermore, the hair care compositions may contain the usual cationic tensides such as quaternised ammonium compounds e.g. cetyltrimethylammoniumchloride or bromide (INCI: cetrimoniumchloride or bromide), hydroxyethylcetyldimonium phosphate (INCI: Quaternium-44), Luviquat^{®} Mono LS (INCI: Cocotrimoniummethosulfate), poly(oxy-1,2-ethanediyl), (Octadecylnitrilio) tri-2,1-ethandiyl) tris-(hydroxy)-phosphate (INCI Quaternium-52).

For special effects typical conditioning agents may be combined with the µ-opioid receptor antagonists within the hair car preparations such as the previously mentioned cationic polymers named polyquaternium (INCI), especially copolymers of vinylpyrrolidone/ N-vinylimidazolium salts (Luviquat^{®} FC, Luviquat^{®} HM, Luviquat^{®} MS, Luviquat^{®} Ultracare), copolymers of N-vinylpyrrolidone/ dimethylaminoethylmethacrylate quaternised with diethylsulfate (Luviquat^{®} PQ 11), copolymers of N-cationic cellulose derivatives (Polyquaternium-4 and-10), acrylamidecopolymers (Polyquatemium-7). Furthermore protein hydrolysates may be used as well as conditioning agents on silicone basis such as polyarylsiloxane, polyarylalkylsiloxane, polyethersiloxane or silicone resins. Other suitable silicone compounds are dimethicone copolyol (CTFA) and amino functionalised silicone derivatives such as amodimethicone (CTFA).

Furthermore cationic guar derivatives such as guarhydroxypropyltrimoniumchloride (INCI) may be used.

One or more anionic, neutral, amphoteric or cationic tensides are optionally present in a total amount of at least 0.01 wt. % of the total weight of the composition. Preferably about 0.01 wt. % to about 50 wt. % of the total weight of the composition of the present invention is used. Most preferred, about 0.1 wt. % to about 40 wt. % of one or more tensides are used.

### Oil and fatty components

The lipid phase can advantageously be chosen from mineral oils and mineral waxes; oils such as triglycerides of caprinic acid and/ or caprylic acid or castor oil; oils or waxes and other natural or synthetic oils, in an preferred embodiment esters of fatty acids with alcohols e.g. isopropanol, propyleneglycol, glycerine or esters of fatty alcohols with carbonic acids or fatty acids; alkylbenzoates; and/ or silicone oils.

Exemplary fatty substances which can be incorporated in the oil phase of the emulsion, microemulsion, oleo gel, hydrodispersion or lipodispersion of the present invention are advantageously chosen from esters of saturated and/ or unsaturated, linear or branched alkyl carboxylic acids with 3 to 30 carbon atoms, and saturated and/ or unsaturated, linear and/ or branched alcohols with 3 to 30 carbon atoms as well as esters of aromatic carboxylic acids and of saturated and/ or unsaturated, linear or branched alcohols of 3-30 carbon atoms. Such esters can advantageously be selected from octylpalmitate, octylcocoate, octylisostearate, octyldodecylmyristate, cetearylisononanoate, isopropylmyristate, isopropylpalmitate, isopropylstearate, isopropyloleate, n-butylstearate, n-hexyllaurate, n-decyloleate, isooctylstearate, isononylstearate, isononylisononanoate, 2-ethyl hexylpalmitate, 2-ethylhexyllaurate, 2-hexyldecylstearate, 2-octyldodecylpalmitate, stearylheptanoate, oleyloleate, oleylerucate, erucyloleate, erucylerucate, tridecylstearate, tridecyltrimellitate, as well as synthetic, half-synthetic or natural mixtures of such esters e.g. jojoba oil.

Other fatty components suitable for hair care compositions include polar oils such as lecithins and fatty acid triglycerides, namely triglycerol esters of saturated and/ or unsaturated, straight or branched carboxylic acid with 8 to 24 carbon atoms, preferably of 12 to 18 carbon-atoms whereas the fatty acid triglycerides are preferably chosen from synthetic, half synthetic or natural oils (e.g. cocoglyceride, olive oil, sun flower oil, soybean oil, peanut oil, rape seed oil, sweet almond oil, palm oil, coconut oil, castor oil, hydrogenated castor oil, wheat oil, grape seed oil, macadamia nut oil and others); apolar oils such as linear and/ or branched hydrocarbons and waxes e.g. mineral oils, vaseline (petrolatum); paraffins, squalane and squalene, polyolefins, hydrogenated polyisobutenes and isohexadecanes, favored polyolefins are polydecenes; dialkyl ethers such as dicaprylylether; linear or cyclic silicone oils such as preferably cyclomethicone (octamethylcyclotetrasiloxane; cetyldimethicone, hexamethylcyclotrisiloxane, polydimethylsiloxane, poly(methylphenylsiloxane) and mixtures thereof.

Other fatty components which can advantageously be incorporated in hair care compositions are isoeikosane; neopentylglycoldiheptanoate; propyleneglycoldicaprylate/ dicaprate; caprylic/ capric/ diglycerylsuccinate; butyleneglycol caprylate/ caprate; C₁₂₋₁₃-alkyllactate; di-C₁₂-₁₃-alkyltartrate; triisostearin; dipentaerythrityl hexacaprylate/ hexacaprate; propyleneglycolmonoisostearate; tricaprylin; dimethylisosorbid. Especially beneficial is the use of mixtures C₁₂₋₁₅-alkylbenzoate and 2-ethylhexylisostearate, mixtures C₁₂₋₁₅-alkylbenzoate and isotridecylisononanoate as well as mixtures of C₁₂₋₁₅-alkylbenzoate, 2-ethylhexylisostearate and isotridecylisononanoate.

The oily phase of the formulation of the present invention can also contain natural vegetable or animal waxes such as bees wax, china wax, bumblebee wax and other waxes of insects as well as shea butter and cocoa butter.

### Silicone oils

Suitable silicone oils are e.g. dimethylpolysiloxane, diethylpolysiloxane, diphenylpolysiloxane, cyclic siloxanes, poly(methylphenylsiloxanes) as well as amino-, fatty acid-, alcohol-, polyether-, epoxy-, fluoro-, glycoside-, and/ or alkyl modified silicone compounds which are liquid or solid at room temperature and mixtures thereof. The number average molecular weight of the dimethicones and poly(methylphenylsiloxanes) is preferably in the range of 100 to 150000 g/ mol. Preferred cyclic siloxanes comprise 4- to 8- membered rings which are for example commercially available as cyclomethicones. An oil or fatty component is optionally present in an amount of about 0 wt. % to about 50 wt. % of the total weight of the product. The preferred amount of an oil or fatty component is about 1 wt. % to about 25 wt. %, and most preferably about 3 wt. % to about 20 wt. %.

### Moisturizing agents

A moisturizing agent may be incorporated into a product to maintain hydration or rehydrate the hair. Moisturizers that prevent water from evaporating from skin or hair by providing a protective coating are called emollients. Additionally an emollient provides a softening or soothing effect on the skin or hair surface. Preferred emollients include mineral oils, lanolin, petrolatum, caprylic/ capric triglycerides, cholesterol, silicones such as dimethicone, cyclomethicone, almond oil, jojoba oil, avocado oil, castor oil, sesame oil, sunflower oil, coconut oil and grape seed oil, cocoa butter, olive oil, aloe extracts, fatty acids such as oleic and stearic, fatty alcohols such as cetyl and hexadecyl (ENJAY), diisopropyl adipate, hydroxybenzoate esters, benzoic acid esters of C₉₋₁₅-alcohols, isononyl iso-nonanoate, ethers such as polyoxypropylene butyl ethers and polyoxypropylene cetyl ethers, and C₁₂₋₁₅-alkyl benzoates, and mixtures thereof. The most preferred emollients are hydroxybenzoate esters, C₁₂₋₁₅-alkyl benzoates and mixtures thereof. An emollient is optionally present in an amount of about 1 wt. % to about 50 wt. % of the total weight of the product. The preferred amount of emollient is about 2 wt. % to about 25 wt. %, and most preferably about 3 wt. % to about 20 wt. %.

Moisturizers that bind water, thereby retaining it on the skin or hair surface are called humectants. Examples of humectants which can be incorporated into a product are glycerine, propylene glycol, polypropylene glycol, polyethylene glycol, lactic acid, sodium lactate, pyrrolidone carboxylic acid, urea, phospholipids, collagen, elastin, ceramides, lecithin sorbitol, PEG-4, and mixtures thereof. Additional suitable moisturizers are polymeric moisturizers of the family of water soluble and/ or swellable/ and/ or with water gelating polysaccharides such as hyaluronic acid, chitosan and/ or a fucose rich polysaccharide which is e.g. available as Fucogel®1000 (CAS-Nr. 178463-23-5) by SOLABIA S. One or more humectants are optionally present at about 0.1 wt. % to about 10 wt. % in a product, preferably about 0.5 wt. % to about 6 wt. %.

The aqueous phase of the products can contain the usual cosmetic additives such as alcohols, especially lower alcohols, preferably ethanol and/ or isopropanol, low diols or ethylene glycol monoethyl- or monobutylether, propylene glycol monomethyl- or - monoethyl- or-monobutylether, diethylene glycol monomethyl-or monoethylether and analogue products, polymers, foam stabilizers; electrolytes and especially one or more thickeners.

### Thickeners

Thickeners that may be used in formulations to assist in making the consistency of a product suitable include carbomer, siliciumdioxide, magnesium and/ or aluminum silicates, lipid thickeners, e.g. cetyl alcohol, cetyl palmitate (Cutina CP, Cognis Cooperation), glyceryl myristate (Estol 3650, Uniqema), microcrystalline wax (A&E Connock), myristyl alcohol (Lanette 14, Cognis Cooperation), myristyl lactate (Crodamol ML, Croda Chemicals), beeswax (A&E Connock), stearic acid (Lipo Chemicals), stearyl alcohol (Lanette 18, Cognis Cooperation), polysaccharides and their derivatives such as xanthan gum (Keltrol, CP Kelco), hydroxypropyl cellulose (Klucel, Hercules Incorporated), hydroxyethylcellulose (Tylose H, Clariant Corporation), polyacrylamides, selfemulsifying polyacrylamide, e.g. Salcare SC 91, Salcare SC 96 (Ciba Specialty Chemicals), Sepigel 305 (Seppic), acrylate crosspolymers, preferably a carbomer, such as Carbopole^{®} of type 980, 981, 1382, 2984, 5984, ETD 2001, ETD 2050, Ultrez 10, Ultrez 21 (Noveon Inc.), alone or mixtures thereof. Thickeners can be present in an amount of about 0.01 wt. % to about 8 wt. % in the product of the present invention, preferably, 0.05 wt. % to about 5 wt. %.

### Neutralizing agents

Examples of neutralizing agents which may be included in the product to neutralize components such as e.g. an emulsifier or a foam builder/ stabilizer include but are not limited to alkali hydroxides such as a sodium and potassium hydroxide; organic bases such as diethanolamine (DEA), triethanolamine (TEA), aminomethyl propanol, and mixtures thereof; amino acids such as arginine and lysine and any combination of any foregoing. The neutralizing agent can be present in an amount of about 0.01 wt. % to about 8 wt. % in the product, preferably, 0.1 wt. % to about 5 wt. %.

### Electrolytes

The addition of electrolytes into the product may be necessary to change the behavior of a hydrophobic emulsifier. Thus, the emulsions/ microemulsions of this invention may contain preferably electrolytes of one or several salts including anions such as chloride, sulfate, carbonate, borate and aluminate, without being limited thereto. Other suitable electrolytes can be on the basis of organic anions such as, but not limited to, lactate, acetate, benzoate, propionate, tartrate and citrate. As cations preferably ammonium, alkylammonium, alkali- or alkaline earth metals, magnesium-, iron- or zinc-ions are selected. Especially preferred salts are potassium and sodium chloride, magnesium sulfate, zinc sulfate and mixtures thereof. Electrolytes can be present in an amount of about 0.01 wt. % to about 8 wt. % in the product.

### Insect repellents

Examples of insect repellents which can be used in hair care compositions are for example N,N-diethyl-m-toluamide, 1,2-pentanediol or insect repellent 3535.

### Anti-dandruff agents

Examples of anti-dandruff agents which may be used are climbazole, octopirox and zinc pyrithione.

### Film forming agents

Customary film formers include, for example, chitosan, microcrystalline chitosan, quatemized chitosan, polyvinylpyrrolidone, vinylpyrrolidone/ vinyl acetate copolymers, polymers of quaternary cellulose derivatives containing a high proportion of acrylic acid, collagen, hyaluronic acid and salts thereof and similar compounds.

One or more film forming agents are optionally present at about 0.1 wt. % to about 10 wt. % in a product of the present invention, preferably about 0.2 wt. % to about 6 wt. %.

### Preservatives

Examples of preservatives include Methyl-, Ethyl-, Propyl-, Butylparabens, Benzalkonium Chloride, 2-Bromo-2-nitro-propane-1,3-diol, Dehydroacetic acid, Diazolidinyl Urea, 2-Dichlorobenzyl alcohol, DMDM Hydantoin, Formaldehyde solution, Methyldibromoglutaronitrile, Phenoxyethanol, Sodium Hydroxymethylglycinate, Imidazolidinyl Urea, Triclosan and further substance classes listed in the following reference: K. F. De Polo-A short textbook of cosmetology, Chapter 7, Table 7-2,7-3, 7-4 and 7-5, p. 210-219.

### Bacteria-inhibitina agents

Typical examples of bacteria-inhibiting agents are preservatives that have a specific action against gram-positive bacteria, such as 2,4,4'-trichloro-2'-hydroxydiphenyl ether, chlorhexidine (1,6-di (4-chlorophenyl-biguanido) hexane) or TCC (3,4,4'-trichlorocarbanilide). A large number of aromatic substances and ethereal oils also have antimicrobial properties. Typical examples are the active ingredients eugenol, menthol and thymol in clove oil, mint oil and thyme oil. A natural deodorizing agent of interest is the terpene alcohol farnesol (3,7,11-tri-methyl-2,6,10-dodecatrien-1-ol), which is present in lime blossom oil. Glycerolmonolaurate has also proved to be a bacteriostatic agent. The amount of the additional bacteria-inhibiting agents present is usually from 0.1 to 2 wt. %, based on the solids content of the preparations.

### Polymers

The compositions may comprise additional polymers which are different from the µ-opioid receptor antagonists in order to establish the desired properties. For this purpose all anionic-, cationic-, amphoteric- or neutral polymers may be used.

Examples for anionic polymers are homo- and copolymers of acrylic acid or methacrylic acid and the salts thereof; copolymers of acrylic acid and acryl amide and the salts thereof; sodium salts of polyhydroxy carbonic acids, water soluble or water dispersable polyester; polyurethanes such as Luviset pur^{®} of BASF and polyureas. Especially suitable are copolymers of t-butyl acrylate, ethylacrylate, methyl acrylic acid (e.g. Luvimer^{®} 100P); copolymers of ethylacrylate and methacrylic acid (e.g. Luviflex^{®} Soft); copolymers of N-tert.-butyl-acryl amide, ethylacrylate and acrylic acid (Ultrahold^{®}, strong); copolymers of vinylacetate, crotonic acid and eventually other vinylic esters (e.g. Luviset^{®} grades); copolymers of maleic acid anhydride; eventually with alcohols reacted anionic polysiloxanes e.g. carboxy functionalized, t-butylacrylate, methacrylic acid (Luviskol^{®} VBM); copolymers of acrylic acid and methacrylic acid with hydrophobic monomers such as C₄-C₃₀-alkylester of methacrylic acid, C₄-C₃₀-alkylvinylester, C₄-C₃₀-alkylvinylether and hyaluronic acid. Examples of anionic polymers are also vinylacetate/ crotonic acid copolymers (Resyn^{®} by National Starch or Gafset^{®} by GAF); vinylpyrrolidone/ vinylacrylate copolymers (e.g. Luviflex^{®} by BASF). Other suitable polymers are vinylpyrrolidone/ acrylate terpolymer and sodiumsufonate containing polyamides or sodiumsulfonate containing polyesters.

Additional polymers which can be used in combination with the µ-opioid receptor antagonists comprises Balance^{®}CR (National Starch; Acrylate Copolymer), Balance^{®} 0/55 (National Starch; Acrylate Copolymer), Balances^{®} 47 (National Starch; Octylacrylamide/ Acrylate/ Butylaminoethylmethacrylate-Copolymer), Aquaflex^{®}FX 64 (ISP; Isobutylene/ Ethylmaleimide/ Hydroxyethylmaleimide-Copolymer), Aquaflex^{®}SF-40 (ISP/ National Starch; VP/ vinylcaprolactam/ DMAPA Acrylate Copolymer), Allianz^{®} LT-120 (ISP/ Rohm & Haas; Acrylate/ C1-2 succinate/ hydroxyacrylate copolymer), Aquarez^{®} HS (Eastman; Polyester-1), Diaformer^{®} Z-400 (Clariant; Methacryloylethylbetaine/ Methacrylate-Copolymer), Diaformer^{®} Z-711 (Clariant; Methacryloylethyl N-oxide/ Methacrylate-Copolymer), Diaformer^{®} Z-712 (Clariant; Methacryloylethyl N-oxide/ Methacrylate-Copolymer), Omnirez^{®} 2000 (ISP; Monoethylester from Poly(Methylvinylether/ Maleic Acid in Ethanol), Amphomer^{®} HC (National Starch; Acrylate/ Octylacrylamide-Copolymer), Amphomer^{®} 28-4910 (National Starch; Octylacrylamide/ Acrylate/ Butylaminoethylmethacrylate-Copolymer), Advantage^{®} HC 37 (ISP; Terpolymer of vinylcaprolactam/ N-vinylpyrrolidone/ dimethylamino-ethylmethacrylate), Advantage^{®} LC55 und LC80 or LC A und LC E, Advantage^{®} Plus (ISP; VA/ butylmaleate/ isobornylacrylate copolymer), Aculyne^{®}258 (Rohm & Haas; Acrylate/ Hydroxyesteracrylate-Copolymer), Luviset^{®} P.U.R. (BASF, Polyurethane-1), Luviflex^{®} Silk (BASF), Eastman^{®} AQ 48 (Eastman), Styleze^{®} CC-10 (ISP; VP/ DMAPA Acrylates Copolymer), Styleze^{®} 2000 (ISP; VP/ Acrylates/ Lauryl Methacrylate Copolymer), DynamX (National Starch; Polyurethane-14 AMP-Acrylates Copolymer), Resyn XP (National Starch; Acrylates/ Octylacrylamide Copolymer), Fixomer A-30 (Ondeo Nalco; polymethacrylic acid (and) acrylamidomethyl propanesulfonic acid), Fixate G-100 (Noveon; AMP-Acrylates/ Allyl Methacrylate Copolymer).

Examples of cationic polymers are Polyquaternium (INCI), e.g. copolymers of vinylpyrrolidone/ N-vinylimidazolium salts (Luviquat^{®}FC, Luviquat^{®} HM, Luviquat^{®}MS, Luviquat^{®}Ultracare), copolymers of N-vinylpyrrolidone/ dimethylaminoethylmethacrylate, quatemized with diethylsulfate (Luviquat^{®} PQ 11, INCI: Polyquaternium-11), copolymers of N-vinylcaprolactam/ N-vinyl-pyrrolidone/ N-vinylimidazolium salts (Luviquat^{®}Hold; INCI: Polyquaternium-46); cationic derivatives of cellulose (Polyquaternium-4 and-10), acrylamidocopolymers (Polyquaternium-7), chitosan, cationic starch derivatives (INCI: starch hydroxypropyltrimonium chloride, corn starch modified), cationic guar derivates (INCI: hydroxypropyl guar hydroxypropyltrimonium chloride), cationic sun flower seed derivatives (INCI: sun flower seed amidopropyl hydroxyethyldimonium chloride), copolymers of acrylic acid, acrylamide and methacrylamidopropyltrimoniumchloride (INCI: Polyquaternium-53), Polyquatemium-32, Polyquaternium-28 without being limited thereto. Suitable cationic quaternized polymers are furthermore Merquat^{®} (polymers on the basis of dimethyldiallyl ammoniumchloride), Gafquat^{®} (quaternary polymers formed by reacting polyvinylpyrrolidone with quaternary ammonium compounds); Polymer JR (hydroxyethylcellulose with cationic groups), and cationic polymers on plant basis such as guar polymers, commercially available as Jaguar^{®} grades of Rhodia.

Examples of neutral polymers are polyvinylpyrrolidone, copolymers of N-vinylpyrrolidone and vinylacetate and/ or vinylpropionate, polysiloxane, polyvinylcaprolactam and other copolymers of N-vinylpyrrolidone, copolymers of N-vinylpyrrolidone and alkylacrylate or methacrylate monomers with C₁-C₁₈-alkyl chains, copolymers of polyvinylalcohol and polyalkylenglycole such as Kollicoats^{®} IR (BASF) or copolymers of other vinyl monomers to polyalkyleneglycol, polysiloxane, polyvinylcaprolactam and copolymers with N-vinylpyrrolidone, polyethylenimine and the salts thereof, polyvinylamine and the salts thereof, cellulose derivatives, chitosan, polyasparaginic acid salts and derivatives thereof, polyethylenimine and the salts thereof, polyvinylamine and the salts thereof such as Luviflex^{®} Swing (partly hydrolysed copolymerisate of polyvinylacetate and polyethyleneglycol by BASF).

Suitable polymers are also non-ionic, water soluble respectively water dispersible polymers or oligomers such as polyvinylcaprolactam, e.g. Luviskol^{®} Plus (BASF), or polyvinylpyrrolidone and copolymers with e.g. vinylesters such as vinylacetate e.g. Luviskol^{®} VA 37 (BASF); polyamide e.g. on the basis of itaconic acid and aliphatic diamines as e.g. described in DE-A-43 33 23.

Examples of amphoteric or zwitterionic polymers are octylacrylamide/ methyl methacrylate/ tert-butylaminoethyl methacrylate/ 2-hydroxypropyl methacrylate copolymers e.g. obtainable under the names Amphomer^{®} (Delft National) and zwitterionic polymers as disclosed, for example, in German Patent Applications DE 39 29 973, DE 21 50 557, DE 28 17 369 and DE 37 08 451. Preferred zwitterionic polymers are acrylamidopropyltrimethylammonium chloride/ acrylic acid or methacrylic acid copolymers and their alkali metal and ammonium salts. Other suitable polymers are methacroylethylbetaine/ methacrylate copolymers, which are obtainable commercially under the name Amersette^{®} (AMERCHOL) and copolymers of hydroxyethyl methacrylate, methyl methacrylate, N,N-dimethylaminoethyl methacrylate and acrylic acid (Jordapon^{®}); Additional suitable polymers are nonionic, siloxane-containing, water-soluble or - dispersible polymers, e.g. polyether siloxanes, such as Tegopren^{®} (Goldschmidt) or Belsil^{®} (Wacker).

One or more polymers are optionally present at about 0.1 wt. % to about 10 wt. % in a product, preferably about 0.2 wt. % to about 8 wt. %.

### Scents and fragrances

The hair care compositions may contain scents and fragrances comprising at least one, preferably numerous odorant ingredients of natural and/ or synthetic origin. The range of the natural odorants includes, in addition to readily volatile, also moderately and only slightly volatile components. The synthetic odorants embrace representatives from practically all classes of odorant substances.

The following list comprises examples of known odorants which may be used in the compositions according to the invention without being limited thereto: natural products such as tree moss absolute, basil oil, tropical fruit oils (such as bergamot oil, mandarin oil, etc.), mastix absolute, myrtle oil, palmarosa oil, patchouli oil, petitgrain oil, wormwood oil, lavender oil, rose oil, jasmine oil, ylang-ylang oil, etc.;
alcohols: farnesol, geraniol, linalool, nerol, phenylethyl alcohol, rhodinol, cinnamic alcohol, (Zyhex-3-en-1-ol, menthol, a-terpineol, etc.;
aldehydes such as citral, alpha-hexyl cinnamaldehyde, Lilial, methylionone, verbenone, nootkatone, geranylacetone, etc.;
esters such as allyl phenoxyacetate, benzyl salicylate, cinnamyl propionate, citronellyl acetate, decyl acetate, dimethylbenzylcarbinyl acetate, dimethylbenzylcarbinyl butyrate, ethyl acetoacetate, cis-3-hexenyl isobutyrate, cis-3-hexenyl salicylate, linalyl acetate, methyl dihydrojasmonate, styralyl propionate, vetiveryl acetate, benzyl acetate, geranyl acetate,etc.;
lactones such as gamma-undecalactone, delta-decalactone, pentadecanolide, 12-oxahexadecanolide,etc.;
acetals such as Viridine (phenylacetaldehyde dimethylacetal),etc.;
and other components often used in perfumery such as indole, p-mentha-8-thiol-3-one, methyleugenol, eugenol, anethol, etc.

### Colorants

Generally, for the coloration of hair care compositions all substances are suitable which have an absorption in the visible light of electromagnetic radiation (400-800nm) The absorption is often caused by the following chromophores: Azo- (mono-, di-, tris-, or poly-)stilbene-, carotenoide-, diarylmethane-, triarylmethane-, xanthene-, acridine-, quinoline-, methine- (also polymethine-) thiazol-, indamine-, indophenol-, azin-, oxazine-, thiazine-, anthraquinone-, indigo-, phthalocyanin and further synthetic, natural and/ or inorganic chromophores.

FD&C and D&C which can be used in hair care compositions according to the invention are e.g. curcumin, riboflavin, lactoflavin, tartrazine, quinoline yellow, cochenille, azorubin, amaranth, ponceau 4R, erythrosine, red 2G, indigotin, chlorophyll, chlorophyllin, caramel, carbo medicinalis, carotenoids, carotin, bixin, norbixin, annatto, orlean, capsanthin, capsorubin, lycopin, xanthophyll, flavoxanthin, lutein, kryptoaxanthin, rubixanthin, violaxanthin, rhodoxanthin, canthaxanthin, betanin, anthocyans without being limited thereto. Examples of dyes are e.g. inorganic pigments such as iron oxide (iron oxide red, iron oxide yellow, iron oxide black etc.) ultramarines, chromium oxide green or carbon black. Other colorants and dyes which can be used in the compositions according to the invention comprise natural or synthetic organic pigments, disperse dyes which may be solubilized in solvents like direct hair dyes of the HC type, for example HC red No. 3, HC Blue No. 2 and all other hair dyes listed in International Cosmetic Ingredient Dictionary Handbook, 11th edition, 2006) or the dispersion dyes listed in Color Index International Society of Dyers and Colorist, color varnishes (insoluble salts of soluble dyes, like many Ca-, Ba- or Al-salts of anionic dyes), soluble anionic or cationic dyes such as acid dyes (anionic), basic dyes (cationic), direct dyes, reactive dyes or solvent dyes, fluorescent dyes, fluorescein and isothiocyanates.

### Other active ingredients

Active ingredients which might be used additionally in hair care compositions comprise vitamins and their derivatives such as tocopherol, tocopheryl acetate, ascorbic acid, ascorbyl phosphate, vitamin Q, D, and K, retinol, retinal, retinoic acid, retinol acetate, retinol palmitate, biotin, carotinoid derivatives such as beta carotene, lycopene, asthaxanthene, vegetable extracts, antibacterial ingredients, instable amino acids comprising dipeptides, oligopeptides and polypeptides such as methionin, cysteine, cystine, tryptophan, phenylalanine, tyrosine, phenols, polyphenols or flavanoids, bisabolol, allantoin, phytantriol, panthenol, AHA acids, ubichinones such as Coenzym Q 10, ceramides, pseudoceramides, essential oils, plant extracts, deoxyribonucleic acid, protein hydrolysates.

Preferably the hair care compositions are in the form of cosmetic hair-treatment preparations, e.g. hair-washing preparations in the form of shampoos and conditioners, hair-care preparations, e.g. pre-treatment preparations, hair tonics, styling creams, styling gels, pomades, hair rinses, treatment packs, intensive hair treatments e. g. leave-on and rinse-off deep conditioners, hair-structuring preparations, e.g. hair-waving preparations for permanent waves (hot wave, mild wave, cold wave), hair-straightening preparations, liquid hair-setting preparations, hair foams, hairsprays, bleaching preparations, e g. hydrogen peroxide solutions, lightening shampoos, bleaching creams, bleaching powders, bleaching pastes or oils, temporary, semi-permanent or permanent hair colorants, preparations containing self-oxidizing dyes, or natural hair colorants, such as henna or chamomile.

Based on the application the hair care preparations may be in the form of a (aerosol) spray, (aerosol) foam, gel, gel spray, cream, lotion, liquid or a wax. Hair sprays comprise as well aerosol sprays as pump sprays without propellant. Hair foams comprise as well aerosol foams as pump foams without propellant. Hair sprays and hair foams comprise mainly or exclusively water soluble or water dispersible components. If the components used in hair sprays or hair foams according to the invention are water dispersible, then they may be in the form of micro dispersions with particle sizes of usually 1-350 nm, preferably 1-250 nm. The solid content of such preparations is typically in the range of 0.5 to 20 wt. % of the total weight of the preparation. Such micro dispersions normally do not need further emulsifiers or tensides for their stabilization.

An exemplary hair care composition (spray) comprises:
1. 0.005 to 5 wt. % of an opioid receptor antagonist
2. 30 to 99.5 wt. %, preferably 40 to 99 wt. %, of at least one solvent chosen from water, water-miscible solvents and mixtures thereof;
3. 0 to 70 wt. % of propellant;
4. 0.1 to 10 wt. % of at least one water-soluble or water-dispersible hair polymer
5. 0 to 0.3 % by weight of at least one water-insoluble silicone;
6. 0 to 0.5 wt. % of at least one wax, preferably at least one fatty acid amide;
7. customary additives.

The hair care compositions can comprise, as component 4), at least one other water-soluble or water-dispersible hair polymer. Typical hair polymers for use in the present invention are commercially available polymers for hair care such as hair styling or conditioning polymers such as e.g. copolymers of vinyl acetate and crotonic acid, copolymers of methyl vinyl ether and maleic anhydride, copolymers of acrylic acid or methacrylic acid with other monomers, polyurethanes, N-vinylpyrrolidone and silicone polymers.

The content of the hair polymer is generally from about 0.1 to 10 % by weight, based on the total weight of the composition. Here, it is preferable to use water-soluble or water-dispersible polyurethanes which, if desired, additionally comprise siloxane groups in copolymerized form.

The composition can comprise, as component 5), at least one water-insoluble silicone, in particular a polydimethylsiloxane, e.g. the Abil^{®} grades from Goldschmidt. The content of this component is then generally from about 0.0001 to about 2 % by weight, preferably from about 0.001 to about 1% by weight, based on the total weight of the composition. Preference is given to using at least one fatty acid amide, such as, for example, erucamide, as component 6).

The hair care compositions can, where appropriate, additionally comprise an antifoam, e.g. based on silicone. The amount of antifoam is generally up to 0.001 % by weight, based on the total amount of the composition. The compositions according to the invention have the advantage that, on the one hand, they impart the desired hold to the hair and, on the other hand, the polymers are easy to wash out (redispersible). Generally, a natural appearance and shine is imparted to the hair, even when the hair is by its very nature especially thick and/or dark.

In a further embodiment, the hair care compositions comprise:
1. 0.05 to 20 wt. % of at least one hair polymer;
2. 20 to 99.95 wt % of water and/ or alcohol;
3. 0 to 79.5 wt. % of customary additives;
4. 0.005 to 5 wt. % of an opioid receptor antagonist.

The term alcohol refers to all alcohols usually used in cosmetics such as ethanol, n-propanol, isopropanol.

Other ingredients are cosmetic adjuvants and additives such as propellants, anti-foaming agents, surface active ingredients e.g. tensides, emulsifiers, foam former and solubilisators. The used surface active ingredients may be anionic, cationic, amphoteric or neutral. Further ingredients may be preservatives, antioxidants, perfume oils, lipidic refatters, active and/ or caring ingredients such as panthenol, collagen, vitamins, protein hydrolysates, alpha- and beta hydroxyl carbonic acids, stabilisators, pH regulators, opacifiers, colorants, dyes, gel formers, salts, moisturizers, complex formers, viscosity regulators or light screening agents without being limited thereto.

In order to obtain certain properties the hair care compositions may additionally comprise conditioning compounds on silicone basis such as polyalkylsiloxane, polyarylsiloxane, polyarylalkylsiloxane, silicone resins, polyethersiloxane or dimethicone copolyole (CTFA) and amino functionalized silicone compounds such as amodimethicone (CTFA), GP 4 Silicone fluid^{®} and GP 7100^{®} (Genesee), Q2 8220^{®} (Dow Corning), AFL 40^{®} (Union Carbide) or polymers as disclosed in EP-B 852 488.

Other suitable ingredients comprise silicone propfpolymers having a polymeric silicone backbone and non-silicone containing side chains or a non silicone containing polymeric backbone and silicone side chains such as Luviflex^{®} Silk or polymers disclosed in EP-B 852 488.

Typical hair care compositions are styling compositions such as hair sprays and hair foams.

In a particular embodiment these compositions comprise:
1. 0.1 to 10 wt. % of at least one hair polymer;
2. 20 to 99 wt. % water and/ or alcohol;
3. 0 to 70 wt. % of at least one propellant;
4. 0 to 20 wt. % of customary additives;
5. 0.005 to 5 wt. % of an opioid receptor antagonist.

Propellants for hair sprays or aerosol foams are typically used propellants. Preferred are mixtures of propane/ butane, pentane, dimethylether, 1,1-difluoroethane (HFC-152a), carbon dioxide, nitrogen or compressed air.

A typical composition for aerosol foams comprises:
1. 0.1 to 10 wt. % of at least one hair polymer;
2. 55 to 99.8 wt. % water and/ or alcohol;
3. 5 to 20 wt. % of a propellant;
4. 0.1 to 5 wt. % of an emulsifier;
5. 0 to 10 wt. % of customary additives.
6. 0.005 to 5 wt. % of an opioid receptor antagonist

Emulsifiers for aerosol foams may be all conventionally used non-ionic, cationic, anionic or amphoteric emulsifier.

Examples of non-ionic emulsifiers comprise (INCI-nomenclature) Laureths, e.g. Laureth-4; Ceteths, e.g. Ceteth-1, polyethyleneglycolcetylether; ceteareths, e.g. ceteareth-25, polyglycol fatty acid glycerides, hydroxylated lecithins, lactyl esters of fatty acids, alkylpolyglycosides.

Examples of cationic emulsifiers are cetyldimethyl-2-hydroxyethylammonium-dihydrogenphosphate, cetyltrimonium chloride, cetyltrimonium bromide, cocotrimonium-methylsulfate, quaternium-1 to 92 (INCI).

Anionic emulsifiers can be selected from alkylsulfate, alkylethersulfate, alkylsulfonate, alkylarylsulfonate, alkylsuccinate, alkylsulfosuccinate, N-alkylsarkosinate, acyltaurate, acylisethionate, alkylphosphate, alkyletherphosphate, alkylethercarboxylate, alpha-olefinsulfonate, especially the alkali-und earth alkali salts, e.g. sodium, potassium, magnesium, calcium, as well as ammonium- and triethanol amine-salts. The alkylethersulfate, alkyletherphosphate and alkylethercarboxylate may comprise between 1 to 10 ethyleneoxide or propyleneoxide units, preferably 1 to 3 ethyleneoxide-units per molecule.

Hair gels comprise exemplary:
1. 0.1 to 20 wt. % preferably 1 to 10 wt. % of at least one hair polymer;
2. 0 to 10 wt. % of at least one carrier (solvent), selected from C2-C5 alcohols, preferably ethanol;
3. 0.01 to 5 wt. %, preferably 0.2 to 3 wt. % of at least one thickener;
4. 0 to 50 wt. % of a propellant;
5. 0 to 10 wt. %, preferably 0.1 to 3 wt. % of a styling polymer different to 1.), preferably a water soluble non-ionic polymer;
6. 0 to 1 wt. % of at least one refatter, preferably selected from glycerine and glycerine derivatives;
7. 0 to 30 wt. % of other customary additives e.g. a silicone component
8. 0.005 to 5 wt. % of an opioid receptor antagonist,
9. water ad 100 wt. %

An exemplary styling gel may comprise:
1. 0.1 to 10 wt. % of a hair polymer;
2. 60 to 99.85 wt. % of water and/ or alcohol;
3. 0.05 to 10 wt. % of a gel former;
4. 0 to 20 wt. % of other customary additives.
5. 0.005 to 5 wt. % of an opioid receptor antagonist,

As gel formers, all typical cosmetic gel formers can be used such as slightly cross linked polyacrylic acid e.g. Carbomer (INCI), cellulose derivatives, polysaccarides e.g. xanthan gum, caprylic/ capric triglyceride (INCI), sodiumacrylate-copolymers, polyquaternium-32 (and) paraffinum liquidum (INCI), sodiumacrylate-copolymers (and) paraffinum liquidum (INCI) (and) PPG-1 trideceth-6, polyquatemium-37 (and) propyleneglycoldicapratdicarylate (and) PPG-1 trideceth-6, polyquaternium-7, polyquatemium-44.

Typical shampoo preparations may comprise:
1. 0.05 to 10 wt. % of a hair polymer;
2. 25 to 94.95 wt. % of water;
3. 5 to 50 wt. % of surfactant;
4. 0 to 5 wt. % of an additional conditioning agent;
5. 0 to 10 wt. % other customary additives.
6. 0.005 to 5 wt. % of an opioid receptor antagonist
7. 0 to 5 wt. % opacifiers and/ or pearly gloss-imparting substances

All typically used anionic, non-ionic, amphoteric or cationic tensides may be used within the shampoo preparations.

Exemplary anionic surfactants comprise alkylsulfate, alkylethersulfate, alkylsulfonate, alkylarylsulfonate, alkylsuccinate, alkylsulfosuccinate, N-alkylsarkosinate, acyltaurate, acylisethionate, alkylphosphate, alkyletherphosphate, alkylethercarboxylate, alpha-olefinsulfonate, especially the alkali-und earth alkali salts, e.g. sodium, potassium, magnesium, calcium, as well as ammonium- and triethanol amine-salts. The alkylethersulfate, alkyletherphosphate and alkylethercarboxylate may comprise between 1 to 10 ethyleneoxide or propyleneoxide units, preferably 1 to 3 ethyleneoxide-units per molecule.

Suitable are e.g. sodium laurysulfate, ammonium laury sulfate, sodium laurylethersulfate, ammonium laurylethersulfate, sodium lauroylsarkonisate, sodiumoleylsuccinate, ammonium laurylsulfosuccinate, sodium dodecylbenzolsulfonate, triethanolamidodecylbenzolsulfonate.

Suitable amphoteric surfactants are e.g. alkylbetaine, alkylamidopropylbetaine, alkylsulfobetaine, alkylglycinate, alkylcarboxyglycinate, alkylamphoacetate or propionate, alkylamphodiacetate or dipropionate such as cocodimethylsulfopropylbetaine, laurylbetaine, cocamidopropylbetaine or sodium cocamphopropionate.

Examples of non-ionic surfactants are e.g. reaction products of aliphatic alcohols or alkylphenols with 6 to 20 C-Atoms of a linear or branched alkyl chain with ethyleneoxide and/ or propyleneoxide. The amount of alkyleneoxide is about 6 to 60 mol to one mol alcohol. Furthermore alkylaminoxide, mono- or dialkylalkanolamide, fatty esters of polyethylene glycols, alkylpolyglycosides or sorbitan ester are suitable for the incorporation of hair care compositions according to the invention.

Furthermore, the shampoo preparations may contain the usual cationic surfactants such as quaternised ammonium compounds e.g. cetyltrimethylammoniumchloride or bromide (INCI: cetrimoniumchloride or bromide), hydroxyethylcetyldimonium phosphate (INCI: Quaternium-44), Luviquat^{®} Mono LS (INCI: Cocotrimoniummethosulfate), poly(oxy-1,2-ethandiyl), (octadecylnitrilio) tri-2, 1-Ethandiyl) tris-(hydroxy)-phosphate (INCI Quatemium-52). Furthermore, cationic guar derivatives such as guarhydroxypropyltrimoniumchloride (INCl) may be used in the shampoo preparations.

In order to provide the formulation a pearlescent appearance or to give the impression of a richer or creamier product, the hair care composition may additionally comprise opacifiers and/ or pearly gloss-imparting substances, such as soaps or salts of carboxylic acids, cationics including cationic polymers, dimethicone (INCI) or amodimethicone (INCI).

Typical conditioner preparations may comprise:
1. 0.05 to 10 wt. % of a hair polymer
2. 5 to 95 wt. % of water
3. 5 to 50 wt. % of surfactant
4. 0 to 5 wt. % of an additional conditioning agent
5. 0 to 10 wt. % other customary additives
6. up to 20 wt. % of an opioid receptor antagonist
   all ingredients adding up to 100 wt. %.

The conditioner preparations may contain usual cationic surfactants and conditioning agents such as quaternised ammonium compounds e.g. cetyltrimethylammoniumchloride or bromide (INCI: cetrimoniumchloride or bromide), stearyl benzyl dimethyl ammonium chloride, distearyldimethylammonium chloride, stearamidopropyldimethylamine, hydroxyethylcetyldimonium phosphate (INCI: Quaternium-44), Luviquat^{®} Mono LS (INCI: Cocotrimoniummethosulfate), poly(oxy-1,2-ethandiyl), (octadecylnitrilio) tri-2, 1-ethandiyl) tris-(hydroxy)-phosphate (INCI Quatemium-52). Furthermore, cationic guar derivatives such as guarhydroxypropyltrimoniumchloride (INCI) may be used in the conditioner preparations.

Other customary additives are for example long chain fatty alcohols such as cetyl alcohol, stearyl alcohol, cetylstearyl alcohol, dimethylstearamine. Furthermore the hair care composition may contain lipids such as dimethicone, amodimethicone, mineral oil, or silicon derivatives such as Dimethicone Copolyol.

Typical temporary hair tinting preparations may comprise:
1. 1 to 95 wt. % of water
2. 0 to 20 wt. % of surfactant
3. 0 to 5 wt. % hair color
4. 0.5 to 10 wt. % hair polymer
5. 0 to 60 wt. % alcohol
6. 0 to 10 wt. % other customary additives
7. up to 20 wt. % of an opioid receptor antagonist
   all ingredients adding up to 100 wt. %.

Typical semi-permanent hair tinting preparations may comprise:
1. 1 to 95 wt. % of water
2. 0 to 20 wt. % of surfactants
3. 0 to 20 wt. % hair color
4. 0 to 20 wt. % solvents
5. 0.5 to 10 wt. % hair polymer
6. 0 to 60 wt. % alcohol
7. 0 to 10 wt. % other customary additives
8. 0 to 5 wt % conditioning agent
9. 0 to 10 wt % neutralizing agent to pH 6-10
10. up to 20 wt. % of an opioid receptor antagonist
   all ingredients adding up to 100 wt. %.

Typical solvents in semi-permanent hair tinting preparations are propylene glycol (INCI), benzyl alcohol, glycol ether such as methoxyisopropanole, diethyleneglycolmonoethylether.

Other customary additives are for example cationic surfactants and conditioning agents such as quaternised ammonium compounds e.g. cetyltrimethylammoniumchloride or bromide (INCI: cetrimoniumchloride or bromide), stearyl benzyl dimethyl ammonium chloride, distearyldimethylammonium chloride, stearamidopropyldimethylamine, hydroxyethylcetyldimonium phosphate (INCI: Quaternium-44), Luviquat^{®} Mono LS (INCI: Cocotrimoniummethosulfate), poly(oxy-1,2-ethandiyl), (octadecylnitrilio) tri-2, 1-ethandiyl) tris-(hydroxy)-phosphate (INCI Quatemium-52). Furthermore, cationic guar derivatives such as guarhydroxypropyltrimoniumchloride (INCI) may be used in semi-permanent hair tinting preparations. Furthermore long chain fatty alcohols such as cetyl alcohol, stearyl alcohol, cetylstearyl alcohol, dimethylstearamine. Furthermore the hair care composition may contain lipids such as dimethicone, amodimethicone, mineral oil, or silicon derivatives such as dimethicone copolyol (INCI).

Typical neutralizing agents are aminomethyl propanol (INCI), monoethanolamine or ammonium hydroxide.

Typical permanent hair tinting preparations may comprise:
Part I:
   1. 1 to 90 wt. % of water;
   2. 0 to 60 wt. % of surfactants
   3. 0 to 30 wt. % emulsifiers
   4. 0 to 30 wt. % soaps
   5. 0 to 20 wt. % hair color
   6. 0 to 20 wt. % solvents
   7. 0 to 10 wt. % cationic polymers
   8. 0 to 20 wt. % alcohol
   9. 0 to 20 wt. % viscosity regulating agents
   10. 0 to 10 wt. % other customary additives
   11. 0 to 5 wt % additional conditioning agent
   12. 0 to 10 wt % alkalizing agents to pH 8-11.5
   13. 0 to 3 wt. % reduction agent such as sodium bisulfit
   14. up to 20 wt. % of an opioid receptor antagonist
      all ingredients adding up to 100 wt. %.
Part II:
   1. 0 to 12 wt. % hydrogen peroxide
   2. 0 to 20 wt. % surfactants / emulsifiers
   3. 0 to 5 wt. % moisturizing agent
   4. 0 to 1 wt. % hydrogen peroxide stabilizing agent
   5. 0 to 5 wt. % neutralizing agent
   6. 57 to 100 wt. % water
Part I:
   Typical cationic surfactants and conditioning agents for permanent hair tinting preparations are e.g. quaternized ammonium compounds e.g. cetyltrimethylammoniumchloride or bromide (INCI: cetrimoniumchloride or bromide), stearyl benzyl dimethyl ammonium chloride, distearyldimethylammonium chloride, stearamidopropyldimethylamine, hydroxyethylcetyldimonium phosphate (INCI: Quaternium-44), Luviquat^{®} Mono LS (INCI: Cocotrimoniummethosulfate), poly(oxy-1,2-ethandiyl), (octadecylnitrilio) tri-2, 1-ethandiyl) tris-(hydroxy)-phosphate (INCI Quatemium-52). Furthermore, cationic guar derivatives such as guarhydroxypropyltrimoniumchloride (INCI) may be used in permanent hair tinting preparations.

Typical solvents in permanent hair tinting preparations are propylene glycol (INCI), benzyl alcohol, glycol ether such as methoxyisopropanole, diethylenglycolmonoethylether. Typical surfactants are anionic, non-ionic, cationic and amphoteric surfactants as mentioned for shampoo preparations.

Typical emulsifiers are e.g. ethoxylated fatty alcohols.

Typical soaps are e.g. ammonium oleate, monoethanolamin oleate.

Typical alkalizing agents are e.g. ammonium hydroxide, monoethanolamine.

Typical viscosity regulating agents are polymers such carbomer (INCI) and fatty alcohols such as cetearyl alcohol (INCI).

Typical cationic polymer is e.g. polyquaternium-34.

Other customary agents are long chain fatty alcohols such as cetyl alcohol, stearyl alcohol, cetylstearyl alcohol, dimethylstearamine. Furthermore the hair care composition may contain lipids such as dimethicone, amodimethicone, mineral oil, or silicon derivatives such as dimethicone copolyol (INCI).

Typical neutralizing agents are aminomethyl propanol (INCI), monoethanolamine or ammonium hydroxide.

Part II:
Typical surfactants / emulsifiers are ethoxylated fatty alcohols.
Typical moisturizing agents are e.g. glycerine or propylene glycol.
Typical hydrogen peroxide stabilizing agents are e.g. sodium stannate, phenacetin (INCI).
Typical neutralizing agents are e.g. phosphoric acid.

The invention is illustrated further by the Examples which follow without being limited thereto.

### Example 1

Melanocytes are isolated and cultured either from hair follicles or epidermal skin derived from a normal adult scalp biopsy. Follicular and epidermal melanocytes are passaged in T25 plastic culture flasks to maximum 4^{th} to maximize in vivo-like behavior. Cells are seeded into flasks and after 2 days are incubated with test substances added to the cell growth medium. The experimental set-up includes flasks in which the cells are either not treated (control) or incubated for 4 days with the appropriate amount of opioid antagonists, to assess the effect on melanin production.

After 4 days incubation, control and treated cells are collected and melanin is extracted from lysed cells. Melanin is quantified by measuring the spectrophotometric OD at 475 nm and calculating against a standardized curve. The resulting value is standardized against the number of cells counted per treatment and is given as micrograms of melanin per million cells. As control known melanin production inducing references IBMX and full medium are used.

**Table 1:**

| | Melanin content In HFM | Melanin content in EM; |
|---|---|---|
| baseline | 100% | 100% |
| IBMX (Conrol) | 140% | - |
| full medium (Control) | 157% | - |
| 100µM Naloxone | 122% | 54% |
| 1µM Naloxone | 129% | 61% |
| 100µM Naloxonazine | 242% | 25% |
| 1µM Naloxonazine | 120% | 40% |
| beta endorphin (µ-opiate agonist) | 92% | - |

As can be seen from the results in table 1, the opiate antagonist significantly increased the melanin content in HFM, whereas in the case of EM the melanin content is significantly reduced.

Furthermore, the hair follicle cells were analysed for their proliferation and dendricity. Proliferation has been assessed by direct cell counting using hemocytometry. Cell dendricity has been assessed by phase contrast microscopy with representative photo documentation. The results are shown in table 2. It can be seen that the opioate receptor antagonists also have an effect on the proliferation and dentricity further supporting the use of the opiate receptor antagonists for the prevention of the graying of hair and/ or for restoration and/ or maintenance of the natural hair color

**Table 2:**

| | Proliferation of melanocytes | Dentricity |
|---|---|---|
| baseline | 100% | 100% |
| IBMX (Conrol) | 140% | 148% |
| full medium | 120% | 123% |
| 1µM Naloxone | 123% | 110% |
| 100µM Naloxone | | 104% |
| 1µM Naloxonazine | 109% | 105% |

### Example 2

### µ-opioid receptor binding assay

250µg/ml membranes from CHO cells overexpressing the µ-opioid receptor (Perkin Elmer) were incubated with gentle shaking for 2h with 50nM [³H] DAMGO (Amersham) in 50mM Tris HCl pH 7.4 (Sigma), 10mM MgCl₂ (Sigma) and 0.2% BSA (Sigma) in the presence of the negative control DMSO (Fluka) or the test compounds. The incubation mixture was transferred to filter plates (Perkin Elmer), the liquid aspirated through the application of a vacuum from the bottom and the plate washed twice with PBS (Invitrogen). The plate was allowed to dry, 35µl of scintillation cocktail (Ultima Gold, Perkin Elmer) added, sealed and placed on a TopCount (Packard) for scintillation counting.

### µ-opioid receptor GTP-recruitment assay

10µg/ml membranes from CHO cells overexpressing the µ-opioid receptor (Perkin Elmer) were incubated with gentle shaking for 1.5h with 100nM DAMGO (Sigma) in 20mM HEPES pH 7.4 (Sigma), 10mM MgCl₂ (Sigma), 100mM NaCl₂ (Fluka), 1µM GDP (Sigma) and 10nM GTP-Eu (Perkin Elmer) in the presence of the negative control DMSO (Fluka) or the test compounds. The incubation mixture was transferred to filter plates (Perkin Elmer), the liquid aspirated through the application of a vacuum from the bottom and the plate washed twice with PBS (Invitrogen) and allowed to dry. Plates were read in an Analyst reader (LJL Biosystems).

Using the methods outlined above the following µ-opioid receptor antagonists have been identified:
Epigallocatechin 3,5-Digallate (37484-73-4), Irigenol Hexaacetate (103652-04-6), Irigenol ex Iris spp (4935-93-7), Berbamine Hydrochloride (5956-76-3), Quercetagetin (90-18-6), Acetylshikonin (24502-78-1), 2',3',4',3,4-Pentahydroxychalcone (484-76-4), beta,beta-Dimethylacryl shikonin (24502-79-2), 2,3-Dimethoxy-5-methyl-1,4-benzoquinone (605-94-7), 2,3-Dimethoxy-5-methylhydroquinone (3066-90-8), 2,3-Dimethoxy-1,4-benzoquinone (3117-02-0), 2,3-Dimethoxyhydroquinone (52643-52-4), Delphinidin chloride (528-53-0), Aureusidin (38216-54-5), Isocembrol (25269-17-4), Robinetin (490-31-3).

### Example 3 Anti Dandruff Shampoo

| *INCI NOMENCLATURE* | *wt. %* |
|---|---|
| Aqua | Ad 100 |
| Ammonium Laureth Sulphate | 35.00 |
| Ammonium Lauryl Sulphate | 15.00 |
| Glycol Distearate | 1.00 |
| Dimethicone | 1.00 |
| Cetyl Alcohol | 0.50 |
| Cocamide MEA | 3.00 |
| ZPT | 1.00 |
| Guar Hydroxipropyltrimonium Chloride | 0.20 |
| Hydrogenated Polydecene | 1.00 |
| Polyquaternium-10 | 0.10 |
| PEG 7m | 0.50 |
| Trimethylpropane Tricaprylate/Tricaprate | 1.00 |
| Preservative | q.s. |
| Parfum | 0.30 |
| E 104,E 110,E 132 | 0.02 |
| *Opioid receptor antagonists according to the invention* | 0.01 |

Combine all ingredients and mix intensively until a homogeneous solution is obtained. At the end add the water under slow agitation and wait until the foam has disappeared.

### Example 4 Conditioner Shampoo

| *INCI NOMENCLATURE* | *wt. %* |
|---|---|
| Aqua | Ad 100 |
| Sodium Laureth Sulphate | 25.00 |
| Cocamidopropyl Betaine | 5.00 |
| Sodium Chloride | 2.50 |
| Glycol Distearate | 1.00 |
| Glycerin | 2.00 |
| Dimethiconol | 0.50 |
| Parfum | 0.50 |
| Coco-Glucoside | 3.00 |
| Carbomer | 0.10 |
| Arginine | 0.05 |
| Glyceryl Oleate | 0.05 |
| Glyceryl Stearate | 1.00 |
| Guar Hydroxypropyltrimonium Chloride | 0.10 |
| Panthenol | 1.00 |
| Disodium EDTA | 0.05 |
| Preservative | q.s. |
| Hydrolyzed Keratin | 0.10 |
| Citric Acid/ Sodium Hydroxide | q.s |
| *Opioid receptor antagonists according to the invention* | 0.005 |
| E 102, E 110, FD&C blue | 0.01 |

Combine all ingredients and mix intensively until a homogeneous solution is obtained. At the end add the water under slow agitation and wait until the foam has disappeared. Than add carefully the thickening agent like sodium chloride.

### Example 5 Shampoo with plant extracts

| *INCI NOMENCLATURE* | *wt.* % |
|---|---|
| Aqua | Ad 100 |
| Sodium Laureth Sulfate | 25.00 |
| Lauryl Glucoside | 10.00 |
| Cocamidopropyl Betaine, | 5.00 |
| Propylene Glycol | 2.0 |
| Parfum | 1.25 |
| Sodium Citrate | 0.25 |
| Sodium Benzoate | 0.20 |
| Panthenol | 1.00 |
| Sodium Formate | 0.20 |
| Polyquaternium-10 | 0.20 |
| Hydroxypropyl Guar Hydroxypropyltrimonium Chloride | 0.05 |
| PEG-35 Castor Oil | 1.00 |
| Maris Sal | 1.25 |
| Polysorbate 20 | 1.00 |
| Tocopheryl Acetate | 0.20 |
| Prunus Armeniaca (Apricot) Fruit Extract | 0.20 |
| Echinacea Purpurea Extract | 0.05 |
| Retinyl Palmitate | 0.05 |
| Tocopherol | 0.05 |
| Linoleic Acid | 0.20 |
| Preservative | 1.00 |
| *Opioid receptor antagonists according to the invention* | 0.01 |
| CI 77891 | 0.02 |

Combine all ingredients and mix intensively until a homogeneous solution is obtained. At the end add the water under slow agitation and wait until the foam has disappeared.

### Example 6 Shine Shampoo

| *INCI NOMENCLATURE* | *wt.* % |
|---|---|
| Aqua | Ad 100 |
| Sodium Laureth Sulfate | 15.00 |
| Disodium Cocoamphodiacetate | 15.00 |
| Sodium Chloride | 2.00 |
| Glycol Distearate | 1.00 |
| Cocamidopropyl PYL Betaine | 2.00 |
| Laurdimonium Hydroxypropyl Hydrolyzed Wheat Protein, | 1.00 |
| PEG-12 Dimethicone | 1.00 |
| Guar Hydroxypropyltrimonium Chloride | 0.05 |
| Hydrolyzed Wheat Protein | 0.20 |
| Laureth-4 | 1.00 |

| | |
|---|---|
| PEG-7 Glyceryl Cocoate | 2.00 |
| Hydrogenated Castor Oil | 1.00 |
| Laureth-2 | 0.50 |
| PEG-55 Propylene Glycol Oleate, | 2.00 |
| Propylene Glycol | 2.00 |
| Mica | 0.20 |
| Citric Acid | 0.01 |
| Parfum | 1.00 |
| E 110, E 104, E 122 | 0.05 |
| *Opioid receptor antagonists according to the invention* | 0.05 |

Combine all ingredients and mix intensively until a homogeneous solution is obtained. At the end add the water under slow agitation and wait until the foam has disappeared. Than add carefully the thickening agent like sodium chloride.

### Example 7 Hydrating Shampoo for Color Protection

| | *INCI Nomenclature* | *wt. %* |
|---|---|---|
| 1 | Sodium Laureth Sulfate | 45.00 |
| | Polysilicone-15 | 0.30 |
| | Methylchloroisothiazolinone & Methylisothiazolinone | 0.10 |
| | Panthenol | 1.00 |
| | PEG-7 Glyceryl Cocoate | 2.00 |
| | Cocamidopropyl Betaine | 10.00 |
| | Glycol Distearate (and) Glycerin (and) Laureth-4 & Cocamidopropyl Betaine | 2.00 |
| | Disodium EDTA | 0.10 |
| | Parfum | 0.80 |
| | Polyquaternium-10 | 0.10 |
| | Decyl Glucoside | 10.00 |
| 2 | Aqua | Ad 100 |
| | *opioid receptor antagonists according to the invention* | 0.01 |
| | Sodium Chloride | 1.50 |
| | PEG-18 Glyceryl Oleate/Cocoate | 1.00 |

Add all ingredients of part 1 and mix intensively until a homogeneous solution is obtained. Add the water under slow agitation and wait until the foam has disappeared. Than add carefully the thickening agent like Sodium Chloride or Crothix LVR.

### Example 8 Extra Shine Revitalizinq Hair Cream

| | *INCI Nomenclature* | *wt. %* |
|---|---|---|
| 1 | Simmondsia Chinensis (Jojoba) Seed Oil | 3.00 |
| | Prunus Armeniaca (Apricot) Kernel Oil | 3.00 |
| | Phenyl Trimethicone | 2.00 |
| | C12-15 Alkyl Benzoate | 2.00 |
| | Glyceryl Stearate SE | 2.00 |
| | Polysilicone-15 | 0.50 |
| | Tocopheryl Acetate | 0.50 |
| | Cetearyl Alcohol | 1.60 |
| 2 | Aqua | Ad 100 |
| | *opioid receptor antagonists according to the invention* | 0.005 |
| 3 | Behentrimonium Chloride | 1.00 |
| | Cocodimonium Hydroxypropyl Hydrolyzed Wheat Protein | 0.30 |
| | Propylene Glycol (and) Diazolidinyl Urea (and) Methylparaben (and) Propylparaben | 1.00 |

Heat part 1 and part 2 separately to 65°C under moderate agitation. When both have the same temperature add part 2 into part 1 under agitation. Let cool to 40°C and add part 3 under agitation, homogenize. Cool to ambient temperature.

### Example 9 Hair Repair Treatment

| | *INCI NOMENCLATURE* | *wt. %* |
|---|---|---|
| A | CetearylOctanoate | 0.20 |
| | Phytantriol | 0.10 |
| | PEG-40 Hydrogenated Castor Oil | 2.00 |
| B | Parfum | q.s. |
| | Cocotrimonium Methosulfate | 2.00 |
| C | Aqua | Ad 100 |
| D | Polyquaternium-16 | 2.00 |
| | Dimethicone Copolyol | 1.00 |
| | *opioid receptor antagonists according to the invention* | 0.5 |
| | Parfum | q.s. |
| | Alcohol denat. | 10.00 |
| | Citric Acid | q.s. |

Heat Part A to 70°C. Add part B to part A under stirring. Add the mixture to part C and homogenize. Add part D and let cool down under moderate agitation.

### Example 10 Color Balm

| | *INCI NOMENCLATURE* | *wt. %* |
|---|---|---|
| A | Ceteareth-6, Stearyl Alcohol | 1.50 |
| | Ceteareth-25 | 1.50 |
| | Cetearyl Alcohol | 3.00 |
| | Cetearyl Octanoate | 6.00 |
| | Phytantriol | 0.30 |
| B | Polyquaternium-44 | 7.70 |
| | *opioid receptor antagonists according to the invention* | 0.005 |
| | Propylene Glycol | 2.00 |
| | Panthenol | 1.00 |
| | Parfum | q.s. |
| | Aqua | Ad 100 |
| C | C.I. 42510, Basic Violet 14 | 0.05 |
| | C.I. 12245, Basic Red 76 | 0.08 |
| | Preservative | q.s. |
| | Citric Acid | q.s. |

Heat part A and B separately to 70 °C. Add part A to B and homogenize. Add part C under stirring.

### Example 11 Silky Hair Cocktail

| | *INCI NOMENCLATURE* | | *wt. %* |
|---|---|---|---|
| A | Caprylic/Capric Triglyceride (and) Acrylates Copolymer | | 3.00 |
| | Dimethicone Copolyol | | 0.50 |
| | Dimethicone Copolyol | | 2.00 |
| | Cyclomethicone (and) Dimethiconol | | 3.00 |
| | Amodimethicone (and) Cetrimonium Chloride (and) Trideceth-10 | | 2.00 |
| | Phenyl Trimethicone | | 2.00 |
| | Macadamia (Ternifloria) Nut Oil | | 1.00 |
| | Tocopheryl Acetate | | 0.50 |
| | PEG-40 Hydrogenated Castor Oil | | 1.00 |
| | Parfum | | q.s. |
| B | Aqua | | Ad 100 |
| | Aminomethyl Propanol | | 0.46 |
| | *opioid receptor antagonists according to the invention* | | 0.01 |
| | PEG/PPG-25/ | 25 | |
| | Dimethicone/ Acrylates Copolymer | | 4.00 |
| | Preservative | | q.s. |

Heat part A and B separately to 70 °C. Add part A to B and homogenize. Let cool down under stirring.

### Example 12 Oil Sheen Moisturizer

| | *INCI NOMENCLATURE* | *wt. %* |
|---|---|---|
| A | Cetyl Alcohol | 2.00 |
| | PEG-75 Lanolin | 1.00 |
| | Glyceryl Stearate | 4.00 |
| | Ceteareth-25 | 1.00 |
| | Cetearyl Octanoate | 4 |
| B | Glycerin | 10.00 |
| | *opioid receptor antagonists according to the invention* | 0.05 |
| | Propylene Glycol | 2.00 |
| | Cocotrimonium Methosulfate | 1.00 |
| | Trimethylsilylamodimethicone, SM 2115 Octoxynol-40, Isolaureth-6, Glycerin | 1.50 |
| | Polysorbate 20 | 1.00 |
| | Aqua | Ad 100 |
| C | Panthenol | 0.50 |
| | Preservative | q.s. |
| | Parfum | q.s. |
| | Citric Acid | q.s. |

Heat part A and B separately to 70°C. Add part A to B and homogenize. Add part C under stirring.

### Example 13 Setting Cream High Gloss

| | *INCI NOMENCLATURE* | *wt.* % |
|---|---|---|
| A | Cetyl Alcohol | 5.00 |
| | Glyceryl Stearate SE | 10.00 |
| | Isopropyl Myristate | 5.00 |
| | Preservative | q.s. |
| | Dimethicone | 1.00 |
| B | Glycerin | 5.00 |
| | *Opioid receptor antagonists according to the invention* | 0.05 |
| | Disodium EDTA | 0.20 |
| | PVP | 2.00 |
| | Aqua | Ad 100 |
| C | Parfum | q.s. |

Heat part A and B separately to 70°C. Add part A to B and homogenize. Add part C under stirring.

### Example 14 Hair Gel

| *INCI NOMENCLATURE* | *wt.* % |
|---|---|
| Carbomer | 0.50 |
| Aqua | Ad 100 |
| Triethanolamine | 0.70 |
| *opioid receptor antagonists according to the invention* | 0.01 |
| PVP | 5.00 |
| Parfum | q.s. |
| PEG-40 Hydrogenated Castor Oil | q.s. |
| Phenoxyethanol (and) Methylparaben (and) Butylparaben (and) Ethylparaben (and) Propylparaben | 0.10 |
| Tocopheryl Actetate | 0.10 |

Combine all ingredients of part 1 and mix intensively until a homogeneous gel is obtained.

### Example 15 Hair Gel

| *INCI NOMENCLATURE* | *wt. %* |
|---|---|
| *opioid receptor antagonists according to the invention* | 0.1 |
| Polyquaternium-46 | 2.50 |
| Alcohol denat. | 15.00 |
| Aqua | Ad 100 |
| Parfum | 0.10 |
| Glycerin | 0.10 |
| Hydroxyethylcellulose | 2.00 |

Combine all ingredients of part 1 and mix intensively until a homogeneous gel is obtained.

### Example 16 Hair Gel

| *INCI NOMENCLATURE* | *wt. %* |
|---|---|
| *opioid receptor antagonists according to the invention* | 0.005 |
| Corn Starch Modified | 2.00 |
| Chitosan | 0.50 |
| Parfum | q.s. |
| PEG-40 Hydrogenated Castor Oil | q.s. |
| PEG-14 Dimethicone | 0.10 |
| Phenoxyethanol (and) Methylparaben (and) Butylparaben (and) Ethylparaben (and) Propylparaben | 0.10 |
| Aqua | Ad 100 |

Combine all ingredients of part 1 and mix intensively until a homogeneous gel is obtained.

### Example 17 Shower Oil

| | *INCI NOMENCLATURE* | *wt. %* |
|---|---|---|
| 1 | MIPA-Laureth Sulfate (and) Laureth-4 (and) Cocamide DEA | Ad 100 |
| | Olive Oil PEG-7 Esters | 5.00 |
| | Persea Gratissima (Avocado) Oil | 35.65 |
| | *opioid receptor antagonists according to the invention* | 0.1 |
| | Tocopherol | 0.10 |
| | Alcohol denat. | 5.00 |
| | Bisabolol | 0.25 |
| | Panthenol | 2.00 |

Combine all ingredients of part 1 and mix intensively until a homogeneous solution is obtained.

### Example 18 Semi-permanent hair tinting formulation

| | *INCI Nomenclature* | *wt. %* |
|---|---|---|
| 1 | Cetearyl Alcohol | 12.00 |
| | Ceteareth-25 | 5.00 |
| | Glyceryl Stearate SE | 2.50 |
| | Oleth-10 | 2.00 |
| | Cetearyl Ethylhexanoate | 0.75 |
| | Glycol Distearate | 0.50 |
| | Polysorbate 60 | 0.50 |
| 2 | Aqua | Ad 100 |
| | Monoethanolamine | 1.00 |
| | Basic Red 51 | 0.15 |
| | Disodium EDTA | 0.05 |
| | *opioid receptor antagonists according to the invention* | 0.05 |
| 3 | Parfum | 0.50 |
| | Hydrolyzed Wheat Protein | 1.00 |

Heat part 1 and 2 to 70 °C. Add part 2 to part 1 under stirring. Then incorporate part 3.

### Example 19 Permanent hair tinting formulation

### Part I

| *INCI Nomenclature* | *wt. %* |
|---|---|
| A Cetearyl Alcohol | 9.00 |
| Sodium Ceteaeyl Sulfate | 3.00 |
| Glyceryl Stearate | 2.50 |
| Laureth-2 | 2.00 |
| Stearamide MEA-Stearate | 0.75 |
| PEG-5 Cocamide | 0.50 |
| Oleic Acid | 0.50 |
| Hair Dye | 0.30 |
| B Aqua | Ad 100 |
| Ammonium Sulfate | 2.00 |
| Sodium Sulfite | 0.50 |
| Disodium EDTA | 0.05 |
| *Opioid receptor antagonists according to the invention* | 0.1 |
| Ascorbic Acid | 0.50 |
| Ammonium Hydroxide | 2.50 |

### Part II

| | *INCI Nomenclature* | *wt. %* |
|---|---|---|
| A | Cetearyl Alcohol | 6.00 |
| B | Aqua | Ad 100 |
| | Hydrogen Peroxide | 9.00 |
| | Sodium Lauryl Sulfate | 3.00 |
| | Disodium Phosphate | 0.15 |
| | Phosphoric Acid | pH 2.0 |

Heat phase A and B of Part I separately to 70°C. Add phase A to phase B under stirring. Adjust the pH to 11.2.

Heat phase A and B of Part II seperately to 70°C. Add phase A to phase B under stirring. Adjust the pH.

Combine Parts I and II shortly before use.

### Example 20 Pharmaceutical Shampoo

### Part I

| | *INCI Nomenclature* | *wt.* % |
|---|---|---|
| A | Aqua | 50.00 |
| | *Opioid receptor antagonists according to the invention* | 5.00 |
| | Methylcellulose | 0.30 |

### Part II

| | *INCI Nomenclature* | *wt. %* |
|---|---|---|
| A | Sodium Laureth Sulfate | 44.50 |
| | Ethylparaben | 0.20 |

### Dissolve Opioid receptor antagonist in water, add Methylcellulose and stir until dissolved MixEthylparaben with Sodium Laureth Sulfate

### Mix part 1 with part 2

### Example 21 Clear Shampoo

| | *INCI Nomenclature* | *wt. %* |
|---|---|---|
| 1 | Sodium Laureth Sulfate | 50.00 |
| | PEG-7 Glyceryl Cocoate | 3.00 |
| | Cocamidopropyl Betaine | 5.00 |
| | Tocopheryl Acetate | 0.10 |
| | Borago Officinalis Seed Oil (and) Tocopherol (and) Ascorbyl Palmitate | 0.30 |
| | PEG-40 Hydrogenated Castor Oil | 4.00 |
| | Parfum | 0.30 |
| | BHT | 0.05 |
| 2 | Panthenol | 1.00 |
| | Disodium EDTA | 0.10 |
| | Aqua | Ad 100 |
| | Methylchloroisothiazolinone (and) Methylisothiazolinone | 0.10 |
| | *Opioid receptor antagonists according to the invention* | 0.005 |
| 3 | Sodium Chloride | 2.00 |
| | PEG-150 Pentaerythrityl Tetrastearate | 3.00 |

Add all ingredients of part 1) and part 2) and mix intensively until a homogeneous solution is obtained. Then, add the water under slow agitation and wait until the foam has disappeared. Finally, add carefully the thickening agent like Sodium Chloride or Crothix LVR.

### Example 22 Hydrating Shampoo

| | *INCI Nomenclature* | wt. % |
|---|---|---|
| 1 | Sodium Laureth Sulfate | 45.00 |
| | Ethylhexyl Methoxycinnamate | 0.30 |
| | Methylchloroisothiazolinone (and) Methylisothiazolinone | 0.10 |
| | Panthenol | 1.00 |
| | PEG-7 Glyceryl Cocoate | 2.00 |
| | Cocamidopropyl Betaine | 10.00 |
| | Glycol Distearate (and) Glycerin (and) Laureth-4 (and) Cocamidopropyl Betaine | 2.00 |
| | Disodium EDTA | 0.10 |
| | Parfum | 0.80 |
| | Polyquaternium-10 | 0.10 |
| | Decyl Glucoside | 10.00 |
| | Sodium Chloride | 1.50 |
| | *opioid receptor antagonists according to the invention* | 0.005 |
| | PEG-18 Glyceryl Oleate/Cocoate | 1.00 |
| | Aqua | Ad 100 |

Add all ingredients of part 1 and mix intensively until a homogeneous solution is obtained. Add the water under slow agitation and wait until the foam has disappeared. Than add carefully the thickening agent like sodium chloride or Crothix LVR.

## Claims

1. The use of a µ-opioid receptor antagonist for the prevention of the graying of hair and/ or for restoration and/ or maintenance of the natural hair color.

2. The use as in claim 1, wherein the µ-opioid receptor antagonist is selected from naloxone, naloxonazine, isocembrol, 2',3',4',3,4-pentahydroxychalcone, aureusidin or 2,3-dimethoxy-5-methylhydroquinone.

3. The use as in claim 2, wherein the µ-opioid receptor antagonist is selected from naloxone, isocembrol, 2',3',4',3,4-pentahydroxychalcone, aureusidin and 2,3-dimethoxy-5-methylhydroquinone.

4. A method of the prevention of the graying of hair and/ or for the restoration and/ or maintenance of native hair color which comprises administering to the scalp or hair of a person in need of such treatment an effective amount of a µ-opioid receptor antagonist.

5. A method as in claim 4, wherein the µ-opioid receptor antagonist is selected from naloxone, naloxonazine, isocembrol, 2',3',4',3,4-pentahydroxychalcone, aureusidin or 2,3-dimethoxy-5-methylhydroquinone.

## Patentansprüche

1. Verwendung eines µ-Opioidrezeptorantagonisten zur Verhinderung des Ergrauens von Haar und/oder zur Wiederherstellung und/oder Erhaltung der natürlichen Haarfarbe.

2. Verwendung nach Anspruch 1, wobei der µ-Opioidrezeptorantagonist aus Naloxon, Naloxonazin, Isocembrol, 2',3',4',3,4-Pentahydroxychalcon, Aureusidin und 2,3-Dimethoxy-5-methylhydrochinon ausgewählt ist.

3. Verwendung nach Anspruch 2, wobei der µ-Opioidrezeptorantagonist aus Naloxon, Isocembrol, 2',3',4',3,4-Pentahydroxychalcon, Aureusidin und 2,3-Dimethoxy-5-methylhydrochinon ausgewählt ist.

4. Verfahren zur Verhinderung des Ergrauens von Haar und/oder zur Wiederherstellung und/oder Erhaltung der ursprünglichen Haarfarbe, bei dem man an die Kopfhaut oder das Haar einer einer solchen Behandlung bedürftigen Person eine wirksame Menge eines µ-Opioidrezeptorantagonisten verabreicht.

5. Verfahren nach Anspruch 4, wobei der µ-Opioidrezeptorantagonist aus Naloxon, Naloxonazin, Isocembrol, 2',3',4',3,4-Pentahydroxychalcon, Aureusidin und 2,3-Dimethoxy-5-methylhydrochinon ausgewählt ist.

## Revendications

1. Utilisation d'un antagoniste du récepteur opioïde µ pour prévenir le grisonnement des cheveux et/ou pour rétablir et/ou maintenir la couleur naturelle des cheveux.

2. Utilisation selon la revendication 1, **caractérisée en ce que** l'antagoniste du récepteur opioïde µ est choisi parmi la naloxone, la naloxonazine, l'isocembrole, la 2',3',4',3,4-pentahydroxychalcone, l'aureusidine ou la 2,3-diméthoxy-5-méthylhydroquinone.

3. Utilisation selon la revendication 2, **caractérisée en ce que** l'antagoniste du récepteur opioïde µ est choisi parmi la naloxone, l'isocembrole, la 2',3',4',3,4-pentahydroxychalcone, l'aureusidine et la 2,3-diméthoxy-5-méthylhydroquinone.

4. Méthode pour prévenir le grisonnement des cheveux et/ou pour rétablir et/ou maintenir la couleur d'origine des cheveux qui comprend l'administration au cuir chevelu ou aux cheveux d'une personne ayant besoin d'un tel traitement d'une quantité efficace d'un antagoniste du récepteur opioïde µ.

5. Méthode selon la revendication 4, **caractérisée en ce que** l'antagoniste du récepteur opioïde µ est choisi parmi la naloxone, la naloxonazine, l'isocembrole, la 2',3',4',3,4-pentahydroxychalcone, l'aureusidine ou la 2,3-diméthoxy-5-méthylhydroquinone.
